Europäisches Patentamt

European Patent Office

Office européen des brevets

(19)

(11) Veröffentlichungsnummer: **0 180 225 B1**

(12) **EUROPÄISCHE PATENTSCHRIFT**

(45) Veröffentlichungstag der Patentschrift: 05.06.91

(21) Anmeldenummer: 85113875.0

(22) Anmeldetag: 31.10.85

(51) Int. Cl.5 **C12N 15/55**, C12N 1 20, C12N 9/84, //(C12N1 20, C12R1:19,C12N9:84,C12R1:19)

(54) **Plasmide zur erhöhten Produktion von Penicillin G-Amidase.**

(30) Priorität: 31.10.84 DE 3439843

(43) Veröffentlichungstag der Anmeldung:
07.05.86 Patentblatt 86/19

(45) Bekanntmachung des Hinweises auf die
Patenterteilung:
05.06.91 Patentblatt 91/23

(84) Benannte Vertragsstaaten:
AT BE CH DE FR GB IT LI LU NL SE

(56) Entgegenhaltungen:
EP-A- 0 107 823
EP-A- 0 120 694

PROC. NATL. ACAD. SCI. USA, Band 80,
21.-25. Januar 1983, Seiten 21-25; H.A. DE
BOER et al.: "The tac promoter: A functional
hybrid derived from the trp and lac promoters"

(73) Patentinhaber: BOEHRINGER MANNHEIM
GMBH
Patentabteilung, Abt. E Sandhofer Strasse
112-132 Postfach 31 01 20
W-6800 Mannheim 31 Waldhof(DE)

(72) Erfinder: Schumacher, Günter, Dr.rer.nat.
Kapellenweg 20
W-8139 Bernried(DE)
Erfinder: Buckel, Peter, Dr. rer.nat.
Pointstrasse 2
W-8139 Bernried(DE)

(74) Vertreter: Weickmann, Heinrich, Dipl.-Ing. et
al
Patentanwälte Dipl.-Ing. H.Weickmann
Dipl.-Phys.Dr. K.Fincke Dipl.-Ing.
F.A.Weickmann Dipl.-Chem. B. Huber Dr.-Ing.
H. Liska Dipl.-Phys.Dr. J. Prechtel Postfach
860820
W-8000 München 86(DE)

CHEMICAL ABSTRACTS, Band 105, Nr. 3, 21. Juli 1986, Seite 195, Zusammenfassung Nr. 19557v, Columbus, Ohio, US; W. BRUNS et al.: "Penicillin acylase: enzyme and gene structure; a prokaryotic peptide with complex proteolytic processing", & EUR. CONGR. BIOTECHNOL., 3rd 1984, 3, 371-6

NUCLEIC ACIDS RESEARCH, Band 14, Nr. 14, 1986, Seiten 5713-3727, IRL Press Ltd, Oxford, GB; G. SCHUMACHER et al.: "Pinicillin acylase from E. coli: unique gene-protein relation"

## Beschreibung

Die Erfindung betrifft ein Plasmid, welches zur Expression von Penicillin-G-Amidase geeignet ist, dieses Plasmid enthaltende Mikroorganismen und ein Verfahren zu seiner Herstellung und seine Verwendung zur Herstellung von Penicillin-G-Amidase.

Penicillin-G-Amidase, auch bezeichnet als Penicillin-Acylase (Penicillin-G-Amidohydrolase, E.C. 3.5.1.11) katalysiert die Spaltung von Penicillin G in 6-Aminopenicillansäure und Phenylessigsäure. 6-Aminopenicillansäure ist die Vorstufe einer großen Reihe von industriell hergestellten semisynthetischen Antibiotika. Es besteht daher ein Bedürfnis nach großen Mengen dieses Enzyms.

Eine Möglichkeit, dies zu erreichen, besteht darin, das Penicillin-G-Amidase-kodierende Gen in ein Plasmid zu klonieren, das in hoher Kopienzahl (ca. 50) in der Zelle vorliegt. Hierbei wurde jedoch nur eine Steigerung um einen Faktor 5 gegenüber dem induzierten Ausgangsstamm ATCC 11105 beobachtet (Mayer, H., Collins, J., Wagner, F., (1979) in: Plasmids of Medical, Environmental and Commercial Importance (Timmis, K.N. und Pühler, A., Eds) 459-469. Elsevier/North-Holland Biomedical Press, Amsterdam). Versuche, das Penicillin-G-Amidase-kodierende Gen in einem geeigneten Expressionsvektor zu einer hohen Syntheseleistung zu bringen, ergaben, daß dies zur Lyse der Wirtszellen führt. Dies zeigt, daß durch erhöhte Expression des Wildtyp Allels des Penicillin-G-Amidase-kodierenden Gens das erstrebte Ziel einer hohen Syntheseleistung nicht zu erreichen ist.

Die Information für ein Enzym ist in der Desoxyribonukleinsäure (DNA) enthalten. Diese DNA wird durch eine DNA-abhängige RNA-Polymerase in mRNA (Boten-Ribonukleinsäure) übersetzt. Die so synthetisierte mRNA wird an den Ribosomen in Protein übersetzt, dabei bestimmen jeweils 3 Nukleotide (Triplett oder Codon) - nach den Gesetzen des genetischen Codes - den Einbau einer bestimmten Aminosäure.

Kontrollbereiche auf DNA-Ebene bestimmen an welcher Stelle ein Strang der DNA in mRNA übersetzt wird (Promotorsequenzen) bzw. an welcher Stelle die Synthese der mRNA gestoppt wird (Terminationssequenzen).

Stop- und Startsequenzen sind ebenso auf der Ebene der Proteinsynthese (Translation) bekannt. Dabei bestimmt im allgemeinen ein ATG (das in f-Methionin übersetzt wird) den Beginn eines Proteins und z. B. ein TAA oder ein TAG das Ende der Translation.

Die Erkenntnisse zur Genexpression sind z. B. beschrieben in B. Lewin, Gene Expression, Vol. 1, 1974, John Wiley + Sons LTD.

Die Neukombination von DNA-Bruchstücken erfolgt bekanntlich in der Art, daß zunächst mit Nukleasen, die bestimmte DNA-Sequenzen "erkennen", die DNA an den diese Sequenzen enthaltenden Stellen geschnitten wird. Da eine große Zahl von Restriktionsendonukleasen bekannt ist, welche jeweils bestimmte DNA-Stellen schneiden, lassen sich DNA-Sequenzen in ganz bestimmter Weise nach Wunsch zerschneiden durch Auswahl der passenden Nukleasen. Solche Restriktionsendonukleasen machen entweder glatte oder überstehende Enden in die doppelsträngige DNA. Die Verknüpfung von glatten bzw. überstehenden Enden erfolgt durch Ligasen genannte Enzyme, meistens mit Hilfe des Enzyms T4-DNA-Ligase.

Der Erfindung liegt nun die Aufgabe zugrunde, das oben erwähnte Problem, das Enzym Penicillin-G-Amidohydrolase in großen Mengen zur Verfügung zu stellen, zu lösen.

Insbesondere ist es ein Ziel der Erfindung, einen Expressions-Vektor von Penicillin-G-Amidohydrolase zu schaffen, der eine hohe Syntheseleistung bringt, ohne zur Lyse der Wirtszellen zu führen.

Gelöst wird diese Aufgabe erfindungsgemäß durch ein zur Expression von Penicillin-G-Amidase geeignetes Plasmid, welches dadurch gekennzeichnet ist, daß es ein inkomplettes Penicillin-G-Amidase-Gen trägt, dem die ersten 78 Basen der translatierten Region am 5' Ende des kompletten Gens fehlen.

Ein Plasmid stellt ein extrachromosomales DNA-Molekül dar. Dieses Molekül trägt die Information, sich selbst zu vermehren (Replikationsursprung) und zusätzlich ein oder mehrere selektionierbare Eigenschaften, z. B. eine Resistenz gegen ein Antibiotikum. Diese Eigenschaften erlauben es, bevorzugt solche Wirtszellen zu erkennen, die das gewünschte Plasmid tragen. Diese Plasmide haben weiterhin die Eigenschaft, daß sie spezifisch mit dem einen oder anderen Restriktionsenzym an bestimmten Stellen gespalten werden können. Anschließend kann nach Zufügen von anderen z. B. mit Restriktionsenzymen gespaltenen DNA-Bruchstücken unter Verknüpfung der Enden ein neues Plasmid geschaffen werden. Eine Anzahl von Plasmiden, die besonders für derartige Manipulationen geeignet sind, ist bereits im Handel erhältlich, z. B. Plasmid pBR 322.

Die Anwendung der Methoden der DNA-Neukombination: DNA-Spaltung und Zusammenfügen von geeigneten DNA-Fragmenten zum neuen Plasmid der Erfindung erfolgt außerhalb der Zelle im Reagenzglas. Das resultierende neue Expressionsplasmid kann durch einen Prozeß, der als Transformation bekannt ist, in eine neue Wirtszelle (Mikroorganismus) überführt werden. Unter der Voraussetzung, daß der DNA-Abschnitt, der das gewünschte Genprodukt kodiert, sich unter der Kontrolle von geeigneten Transkriptions- und Translationsstarts befindet, kann durch diesen Mikroorganismus die Polypeptidsequenz des Enzyms exprimiert werden. Das Genprodukt kann, falls dies notwendig ist, nach Lyse der Wirtszellen und weiteren Reinigungsschritten von anderen Proteinen getrennt erhalten werden.

Die Erfindung basiert auf der überraschenden Erkenntnis, daß das natürliche Penicillin-G-Amidasegen aus einem Genabschnitt, der ein Polypeptid von 846 Aminosäuren codiert und einer vorgeschalteten Promotorsequenz besteht, wobei für die ungenügende Syntheseleistung des kompletten Gens bei Expression durch einen Mikroorganismus nach vorherigem Einbau in ein Plasmid anscheinend vor allem ein Peptid von 54 Aminosäuren, welche die Positionen 236 bis 289 einnehmen, verantwortlich ist. Entfernt man daher die hierfür codierenden Basen 705 bis 867 des Gens, so wird die Expressionshemmung beseitigt.

Man erhält so zwei Genabschnitte, die zwei verkürzte, mit Methionin beginnende Polypeptide kodieren. Diese kürzeren Polypeptide sind, wenn sie zusammen exprimiert werden, miteinander biologisch aktiv und scheinen dem natürlich vorkommenden, aus zwei Untereinheiten bestehenden Enzym zu entsprechen. Das erfindungsgemäße Plasmid kann daher das inkomplette Penicillin-G-Amidase-Gen auch in Form von zwei getrennten Bruchstücken, die durch diese beiden Genabschnitte dargestellt werden, eingebaut enthalten, wobei diese Bruchstücke mit Base Nr. 79 bzw. Nr. 868 beginnen und eine Deletion der kodierenden Basen von 705 bis 867 vorliegt, gezählt vom 5'-Ende des kompletten Gens aus. Da das komplette Gen 2568 Basen enthält, wird im folgenden derjenige Genabschnitt, der mit Base 79 beginnt und mit Base 705 endet und die eine Untereinheit des biologisch aktiven erfindungsgemäß erhaltenen Enzyms kodiert als Genabschnitt der kleinen Untereinheit (alpha) und der mit Base 868 beginnende und mit Base 2541 (einschließlich Stopcodon) oder 2538 (nur translatierte Region) endende Genabschnitt, der die zweite Untereinheit des erfindungsgemäß erhältlichen aktiven Enzyms kodiert, als Genfragment der großen Untereinheit ($\beta$) bezeichnet.

Es wird angenommen, daß beim natürlichen Enzym im Rahmen der sogenannten Proteinreifung erst das biologisch aktive Enzym gebildet wird unter Spaltung des primär erzeugten Proteins und Bildung von zwei Untereinheiten, die dann zum aktiven Enzym zusammentreten. Da die Proteinreifung ein limitierendes Ereignis für die Enzymexpression darstellt, läßt sich durch Anordnung der zwei getrennten Genabschnitte für die alpha- und die $\beta$-Untereinheit eine weitere Erhöhung der Expression von biologisch aktivem Enzym gemäß der Erfindung erzielen. Vor jedem Genabschnitt wird dann ein Startkodon neu eingefügt, in der Regel in Form des Kodons ATG. Neben diesen Kodons müssen am 5'-Ende des jeweiligen "inkompletten" Gens gemäß der Erfindung auch Promotorsequenzen eingefügt sein, die die Übersetzung in mRNA ermöglichen, vorzugsweise der lac-Promotor oder der trp-Promotor. Andere Promotoren können aber ebenfalls verwendet werden.

Das erfindungsgemäße Plasmid enthält vorzugsweise zur besseren Erkennbarkeit von Mikroorganismen, die dieses Plasmid enthalten, wenigstens ein Resistenzgen gegen Chloramphenicol, Tetracyclin oder/und Kanamycin. In einem Medium, das eines der dem jeweils vorhandenen Resistenzgen entsprechendes Antibiotikum enthält, wachsen daher nur solche Mikroorganismen, welche das erfindungsgemäße Plasmid enthalten. Ein derartiges Resistenzgen ist jedoch für die Erfindung an sich nicht notwendig.

Ein weiterer Gegenstand der Erfindung sind Mikroorganismen, die durch einen Gehalt an wenigstens einem Plasmid nach einem der Ansprüche 1 bis 9 gekennzeichnet sind. Enthält dabei das erfindungsgemäße Plasmid nur das Genbruchstück für die große oder für die kleine Untereinheit, so ist zur Bildung des biologisch aktiven Enzyms gemäß der Erfindung entweder erforderlich, daß der Mikroorganismus sowohl ein Plasmid mit dem Genfragment für die kleine Untereinheit als auch ein Plasmid mit dem Genfragment für die alpha-Untereinheit bzw. ein Plasmid, das beide Untereinheiten kodiert, enthält. Wahlweise ist es aber auch möglich, daß ein Mikroorganismus nur ein Plasmid für eine der beiden Untereinheiten enthält. In diesem Falle kann die Züchtung des Mikroorganismus im Gemisch mit einem weiteren Mikroorganismus, der das Plasmid für die andere Untereinheit enthält, erfolgen. Vorzugsweise wird dabei im letzteren Fall für beide Plasmide der gleiche Wirtsmikroorganismus verwendet. Alternativ kann die Züchtung der beiden Mikroorganismen, welche je eine Enzymuntereinheit bilden, getrennt erfolgen. Dann werden erst die Extrakte vermischt unter Bildung eines aktiven Enzyms. Als Wirtsorganismen können die in der Gentechnik allgemein verwendeten Wirtsorganismen, vorzugsweise Derivate des E. coli K12-Stammes, verwendet werden. Besonders gute Ergebnisse wurden mit den E. coli Stämmen 54-2 $\Delta$-(lac, pro) rec A, rpsl, F'lac i$^q$, DSM 3066 und DS 410, min A, min B, rpsl, sup$^*$ , DSM 3065 und DSM 3058 erzielt.

In einer bevorzugten Ausführungsform trägt ein E. coli Stamm 3058 das erfindungsgemäße Plasmid pBT 212. Damit läßt sich nach dem Aufschluß der Zellen und nach 4- bis 8stündiger Inkubation des Zellextraktes die aktive Penicillin-G-Amidase bestehend aus der $\alpha$- und der $\beta$-Untereinheit gewinnen.

Für die Herstellung der erfindungsgemäßen Plasmide verwendet man zweckmäßig bekannte und großenteils handelsübliche Ausgangsplasmide, welche sich speziell für die Expression in bestimmten Mikroorganismen besonders eignen. Beispielsweise eignet sich das Plasmid pBR 322, ein handelsübliches Plasmid, besonders für Expression in allen E. coli Stämmen und wird daher auch im Rahmen der Erfindung vorzugsweise verwendet. Die nachstehende Beschreibung der Herstellung erfindungsgemäßer Plasmide geht daher von Derivaten des Plasmids pBR 322 aus, die ihrerseits teilweise wieder handelsüblich sind oder in bekannter Weise aus diesem Plasmid hergestellt werden können. Jedoch sind für andere Wirtsorganismen als E. coli andere Basisplasmide besser geeignet und wird daher ein nicht zu den E. coli gehörender Wirtsorganismus verwendet, so geht man zweckmäßig von einem Plasmid aus, das in diesem Wirtsorganismus besonders gut exprimiert wird. Derartige Plasmide sind dem Fachmann bekannt und brauchen hier nicht näher erläutert zu werden. Sie sind z. B. beschrieben in ATCC Catalogue of Strains I.

Die Herstellung der erfindungsgemäßen Plasmide erfolgt nach an sich bekannten Methoden, indem geeignete natürlich vorkommende oder durch synthetische Linker erzeugte Restriktionsschnittstellen dazu verwendet werden, das inkomplette Penicillin-G-Amidase-Gen unter die Kontrolle eines funktionstüchtigen Promotors zu bringen. Beispiele für geeignete Promotoren sind der tac-Promotor (F. Amann, J. Brosius, M. Ptashne, Gene 1983) und der lac-Promotor (L. Guarente et al., Cell (1980) 20, 543-553).

Im folgenden wird die Erfindung in Verbindung mit der beigefügten Zeichnung näher beschrieben. In dieser stellen dar:

| | |
|---|---|
| Fig. 1 | die Nukleotid- und Proteinsequenz des kompletten Penicillin-G-Amidohydrolase-Gens, |
| Fig. 2 | im oberen Teil eine schematische Darstellung der für die Klonierung relevanten Restriktionsendonuklease-Schnittstellen und im unteren Teil die relevanten Aminosäuresequenzen der Amino- und Carboxytermini der kleinen und der großen Untereinheiten der gereiften Penicillin-Amidohydrolase, |
| Fig. 3 | schematisch die Konstruktion eines Fusionsproteins, das von Sequenzen der PenicillinG-Amidase und β-Galactosidase codiert wird. |

| | |
|---|---|
| Fig. 4 bis 6 | zeigen die Konstruktion des Plasmids pBT 212, welches zur Expression eines Proteins geeignet ist, welches die Sequenz der Penicillin-G-Amidase enthält und zum aktiven Enzym gereift werden kann. |
| Fig. 7 und 8 | zeigen die Konstruktion des erfindungsgemäßen Plasmids pBT 1000, DSM 3068P, welches die große Untereinheit der Penicillin-G-Amidase codiert und deren Expression bewirken kann. |
| Fig. 9 | die Konstruktion des erfindungsgemäßen Plasmids pBT 702, DSM 3067P, welches die kleine Untereinheit der Penicillin-G-Amidase codiert und deren Expression bewirken kann. |

Die nachstehend beschriebene Herstellung von DNA-Präparationen, Schneiden von DNA mit Restriktionsnukleasen, Zusammenfügen von DNA-Fragmenten und die Bedingungen für die Transformation von Wirtsorganismen sind an sich bekannt und beschrieben beispielsweise in Advanced Bacterial Genetics (1980), Cold Spring Harbor Laboratory von R. W. Davis, D. Botstein und J. R. Roth sowie in Molecular Cloning (1982) Cold Spring Harbor Laboratory von T. Maniatis, E. F. Fritsch und J. Sambrook.

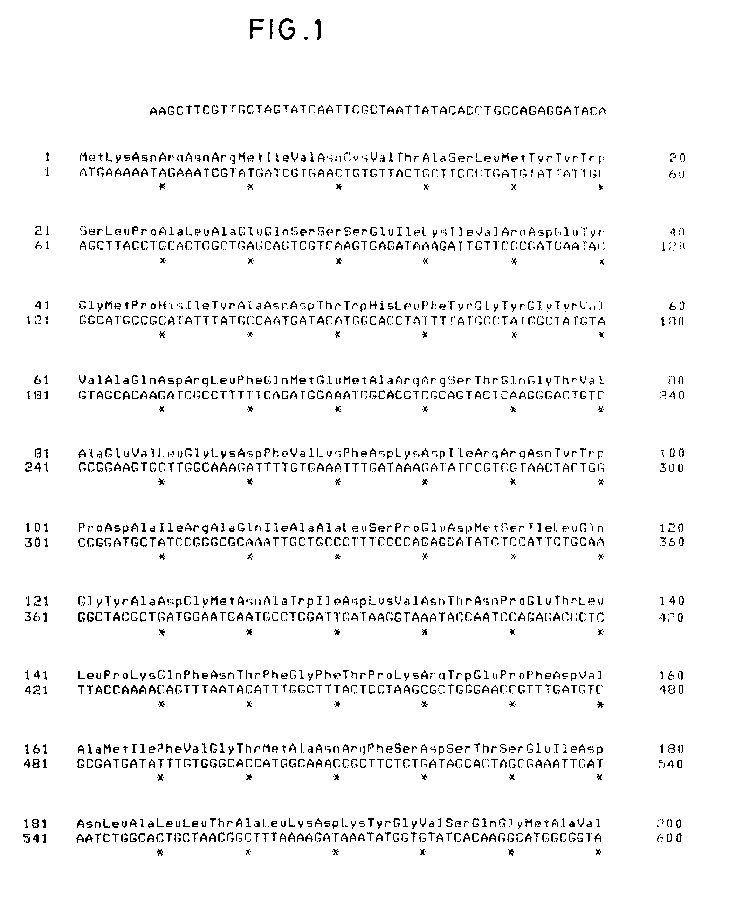

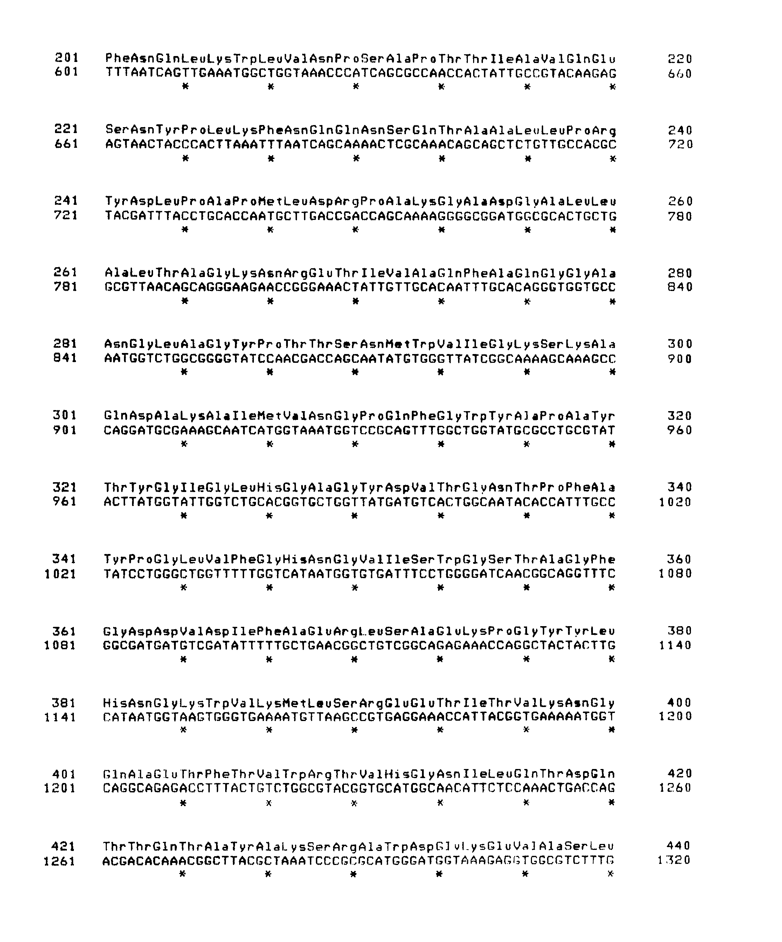

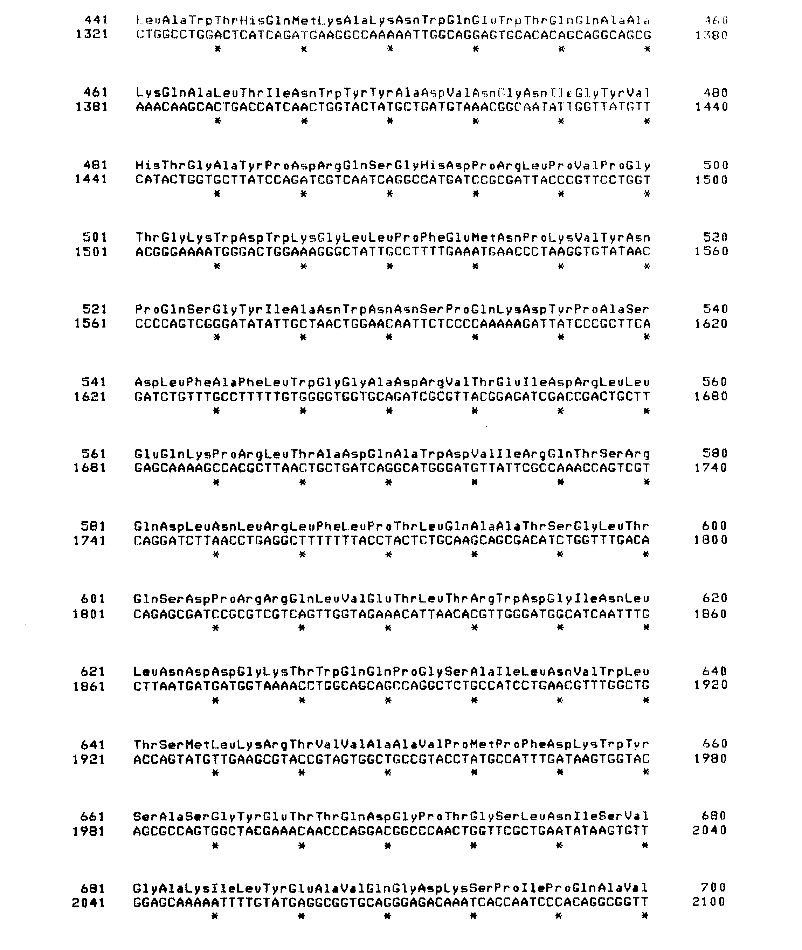

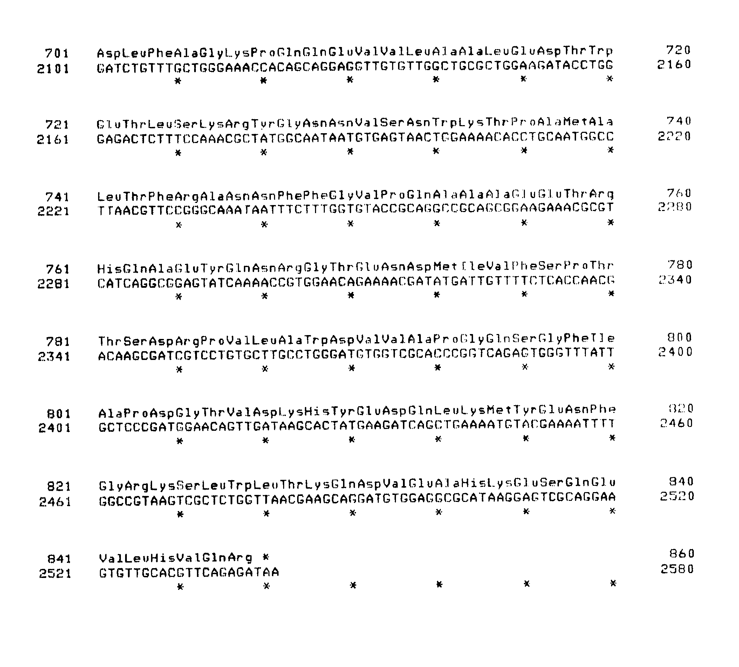

Fig. 1 zeigt das komplette Penicillin-G-Amidase-Gen aus 2538 translatierten Nukleotiden in der Orientierung von 5' nach 3', d. h. der kodierenden mRNA entsprechend. Es bestimmen jeweils 3 (Triplett) der 4 möglichen Nukleotide A, G, C und T eine Aminosäure (obere Reihe). Das im erfindungsgemäßen Plasmid enthaltene inkomplette Penicillin-G-Amidase-Gen beginnt mit dem Triplett GAG in Position 79 bis 81, welches Glu codiert. An dieser Stelle beginnt auch das die kleine Untereinheit codierende Fragment. Das die große Untereinheit des Enzyms kodierende Fragment beginnt in Position 868 mit dem Triplett AGC, welches die Aminosäure Ser codiert und endet bei Position 2538 mit dem Triplett AGA für Arg. Daran schließt sich noch das Stopkodon TAA an, welches im erfindungsgemäßen Plasmid enthalten sein kann aber nicht muß. Das die kleine Untereinheit codierende Genfragment endet mit der Aminosäure Ala mit dem Triplett GCA an Position 703-705.

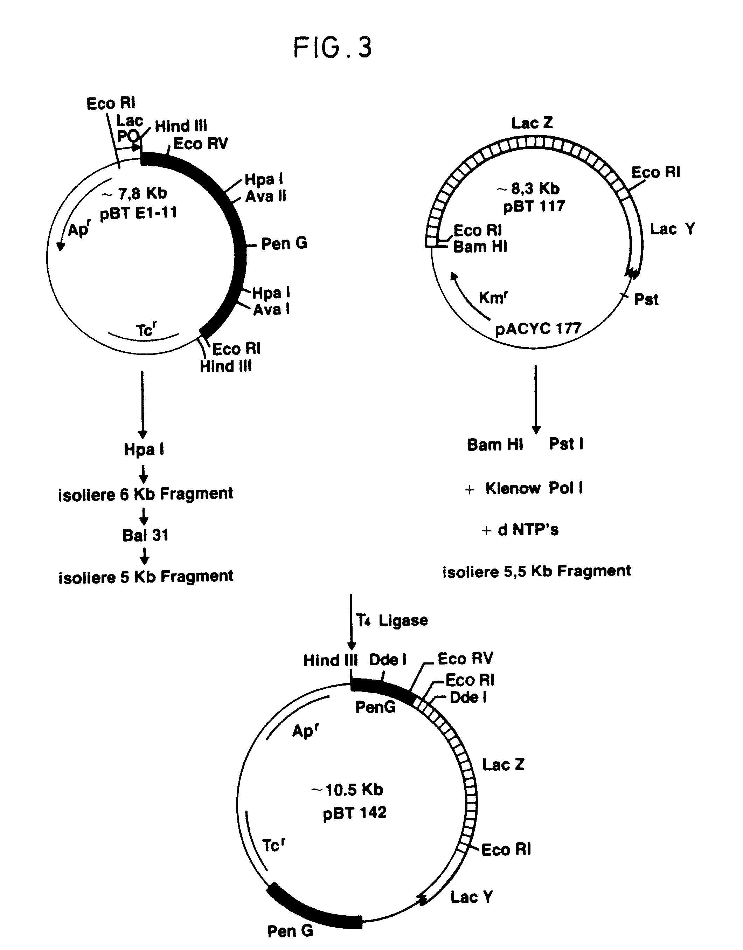

Die erfindungsgemäß dargestellten Plasmide berücksichtigen für die Konstruktion auf DNA-Ebene exakt die aufgrund der Proteinsequenz erhaltenen Carboxy- und Aminotermini der gereiften Untereinheiten. Es ist jedoch bekannt, daß in vielen Fällen Hinzufügen oder Entfernen von Aminosäuren am Carboxy- bzw. am Aminoterminus die Enzymaktivität nicht beeinflußt. So enthält z. B. das in Fig. 3 dargestellte zwischen Penicillin-G-Amidase und β-Galactosidase hergestellte Fusionsprotein neben den Aminosäuren der β-Galactosidase am Aminoterminus noch ca. 120 Aminosäuren der Pen-G-Amidase. Dieses Fusionsprotein zeigt die Enzymaktivität der β-Galactosidase. Daher umfaßt die Erfindung auch derartige Abänderungen, soweit die Enzymaktivität dabei erhalten bleibt.

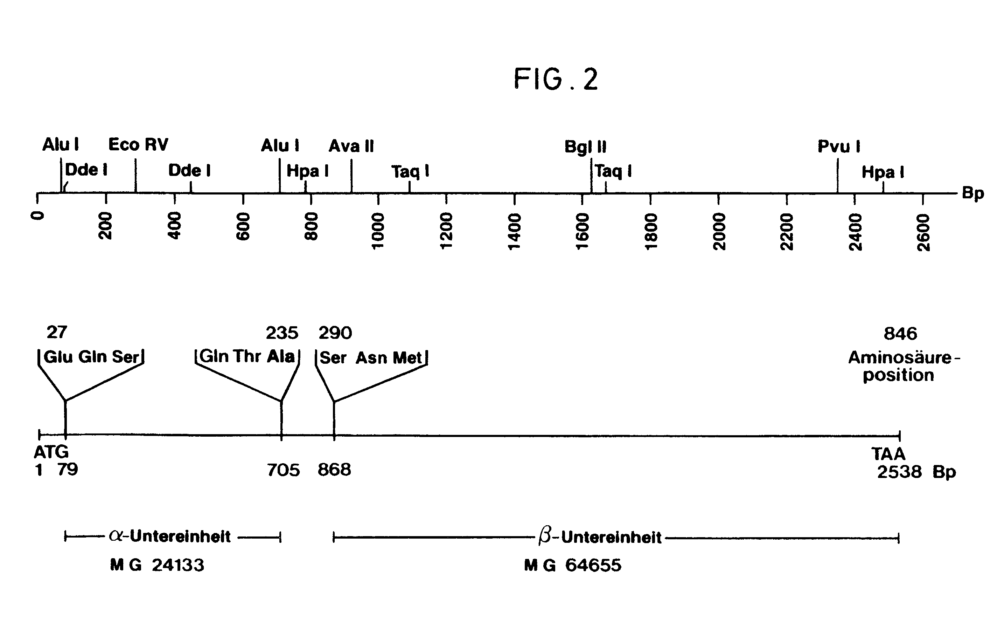

Fig. 2 erläutert dies näher und zeigt unten schematisch jeweils den Beginn der kleinen und der großen Untereinheit, deren Molekulargewicht und das Ende. Oben sind die Spaltstellen für eine Reihe von Nukleasen angegeben, die bei der in den Beispielen beschriebenen Konstruktion ausgenützt werden.

Aus obigem ergibt sich, daß ein erfindungsgemäßes Plasmid die in Fig. 1 gezeigte Nukleotidsequenz, beginnend bei Base Nr. 79, eine der beiden die beiden Bruchstücke kodierenden Fragmente, die mit Base 79 bzw. 868 beginnen und mit Base 705 bzw. 2538 enden oder beide Bruchstücke getrennt voneinander enthält. Erfindungsgemäß werden diese Plasmide zur Herstellung von Penicillin-G-Amidase verwendet, indem man einen Mikroorganismus, der das erfindungsgemäße Plasmid bzw. erfindungsgemäße Plasmide enthält, unter Expression des Enzyms züchtet und das Enzym aus dem Mikroorganismus oder/und der Kulturbrühe gewinnt. Als Mikroorganismus wird hierbei E. coli K12 54-2 oder DS 410 bevorzugt. Letzterer Stamm zeichnet sich durch besondere Stabilität gegen Lyse durch Überproduktion des periplasmatischen Enzyms Penicillin-G-Amidase aus. Die folgenden Beispiele erläutern die Erfindung weiter.

**Beispiel 1**

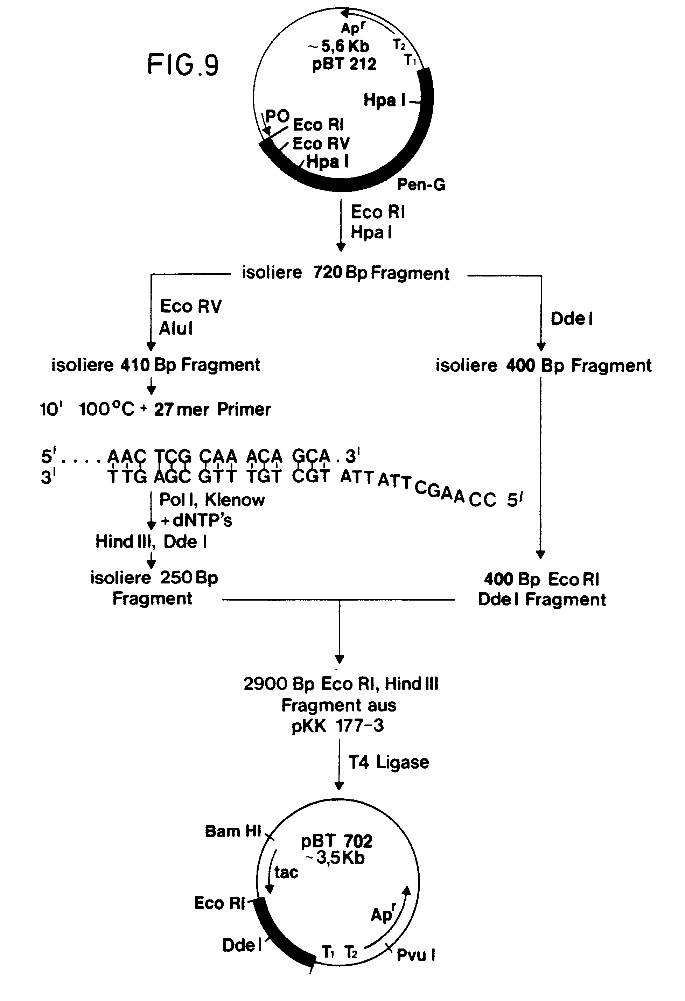

Herstellung des Plasmids pBT 212 (Fig. 3 bis 6 der Zeichnung) und pBT 702 (Fig. 9 der Zeichnung).

Als Ausgangsplasmid wird pBT E1-11, DSM 3061 verwendet. Dieses Plasmid enthält ein ca. 3 Kb großes Penicillin-G-Amidase kodierendes Gen C, welches in Fig. 3 als dicker schwarzer Strich schematisch dargestellt ist). Die Basensequenz dieses Gens ist in Fig. 1 gezeigt. Es kodiert das ebenfalls in Fig. 1 dargestellte enzymatisch aktive Polypeptid von 846 Aminosäuren. Dieses Polypeptid wird beim natürlichen vollständigen Gen dreimal posttranslational gespalten. Das hierbei von den Positionen 1 bis 26 abgespaltene Peptid hat die Größe und die Eigenschaften eines Leader-Peptids (auch als Signalpeptid bezeichnet). Zur erfindungsgemäßen Konstruktion der Penicillin-G-Amidase ohne dieses Leader-Peptid wird vom Plasmid pBT 142, DSM 3059, ausgegangen. Dieses Plasmid kodiert ein Protein, das aminoterminal aus etwa 120 Aminosäuren der Penicillin-G-Amidase besteht und an das distal die β-Galactosidase, beginnend mit der fünften Aminosäure, fusioniert ist.

Plasmid pBT E1-11 (Fig. 3) wird mit der Restriktionsendonuklease Hpa I gespalten und ein 6 Kb-großes Fragment nach Größentrennung in einem niedrigschmelzenden Agarosegel isoliert. Von jedem Ende dieses DNA-Fragments werden mit der Exonuclease Bal 31 ca. 500 Basenpaare (Bp) entfernt.

Plasmid pBT 117, DSM 3063 (Fig. 3) enthält das β-Galaktosidase-Gen ohne Regulationssequenzen, d. h. ohne Promotor und Operator und ohne Startsignal (ATG). Aus Plasmid pBT 117 wird nach Spaltung mit Bam HI und Pst I und Auffüllung bzw. Abspaltung der überstehenden Enden durch DNA-Polymerase I (Klenow-Fragment) in Gegenwart der 4-Desoxy-Ribonukleotid-Triphosphate (dATP, dTTP, dCTP und dGTP) durch Größenfraktionierung in einem niedrig- schmelzenden Agarose-Gel ein 5,5 Kb-großes Fragment isoliert (Fig. 3). 1 $\mu$g dieses Fragments wird mit 0,2 ug des 5 Kb-großen Fragments aus pBT E1-11 über Nacht mit 10 Einheiten T4-Ligase inkubiert. Der Ligierungsansatz wird in den Stamm E. coli K12 54-2 transformiert. Die Selektion erfolgt auf Indikatorplatten, die X-Gal (Miller, J. H., (1972) in: Experiments in Molecular Genetics, Cold Spring Harbor Laboratory, 47-55) und Ampicillin enthalten. Plasmide von β-Galaktosidase-positiven Klonen wurden durch Spaltung mit Eco RI charakterisiert, eines dieser Plasmide ist pBT142 (Fig. 3).

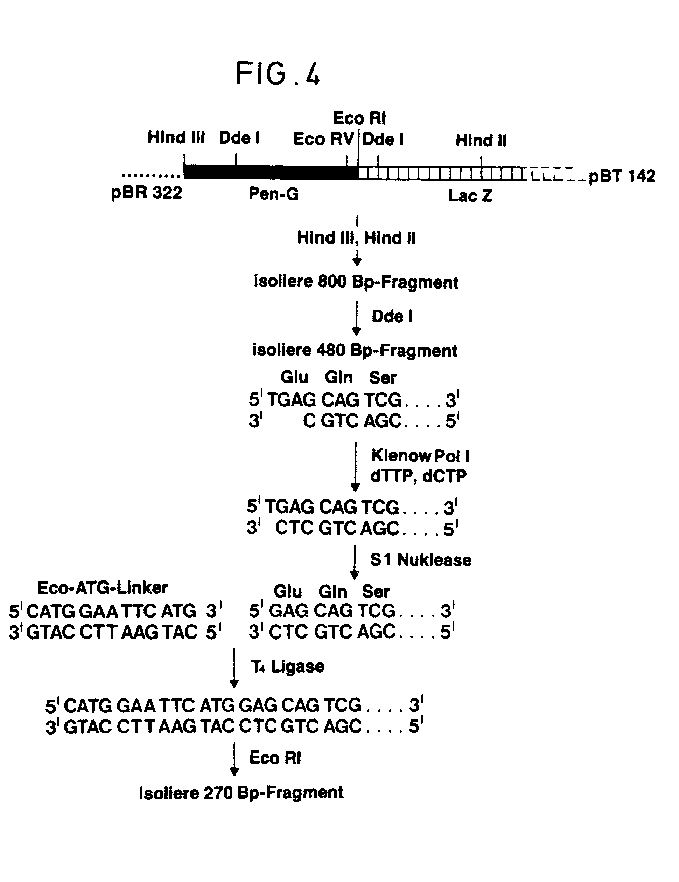

40 $\mu$g des Plasmids pBT142 wurden mit Hind III und Hind II vollständig gespalten und nach Größenfraktionierung ein 800 Bp-Fragment isoliert, dieses Fragment wurde mit Dde I gespalten und so ein 480 Bp-großes Fragment erhalten (Fig. 4). Nach Reinigung dieses Fragments durch Größenfraktionierung in einem niedrigschmelzenden Agarose-Gel, wurde das zurückstehende Ende mit DNA-Polymerase I (Klenow-Fragment) und den Desoxy-Ribonukleotid-Trisphosphaten dTTP und dCTP um 2 Nukleotide aufgefüllt. 0,1 $\mu$g des Fragments werden mit 1 Einheit S1 Nuklease in einem Puffer, der 200 mmol/l NaCl, 50 mmol/l Naacetat pH 4,5, 1 mmol/l ZnSO₄ und 0,5 % Glycerin enthielt, für 30 Minuten bei 30°C inkubiert. Hierdurch wird das am 5'-Ende überstehende Ribonukleotid dTMP abgespalten.

Das resultierende DNA-Fragment enthält ein glattes Ende und das erste Triplett (GAG) kodiert die erste Aminosäure (Glu) der gereiften Form der kleinen Untereinheit der Penicillin-G-Amidase. Um einen Start der Proteinsynthese zu gewährleisten, wird vor das Triplett GAG ein ATG angefügt.

Über die Phosphotriester Methode ( Crea, R., Kaszewski, A., Hiros, T., Itakura, K., (1978) Proc. Natl. Acad. Sci., USA, 75, 5765-5769) wird ein Eco-ATG-Linker mit der Basenfolge

5'CATGGAATTCATG3'
3'GTACCTTAAGTAC5'

synthetisiert.

Der Linker wird mit Polynukleotidkinase phosphoryliert und ein 100-facher Überschuß dieses Linkers wird mit Hilfe von T₄-Ligase an das wie vorher beschriebene glatte Ende des Dde I-Fragments ligiert. Anschließend wird mit 100 Einheiten Eco RI vollständig gespalten und ein 0,27 Kb-Fragment über ein Agarose-Gel isoliert.

Plasmid pKK177-3, DSM 3062wird mit Eco RI und Pst I vollständig gespalten. Über Größenfraktionierung im Agarosegel wird ein 2,9 Kb großes Fragment isoliert.

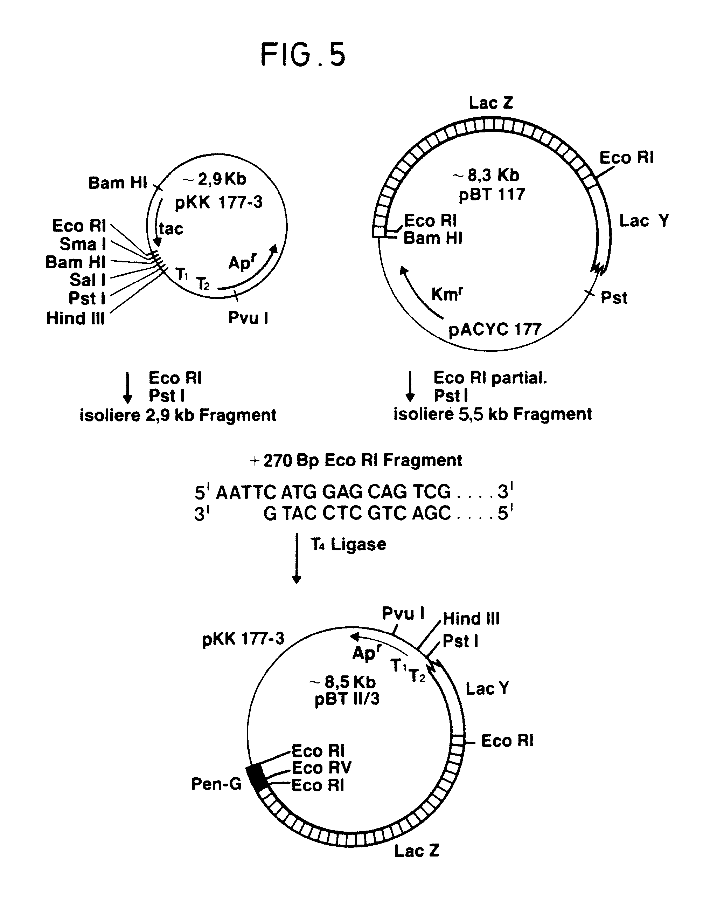

pBT117 wird limitiert mit Eco RI und vollständig mit Pst I gespalten. Durch Größenfraktionierung im Agarosegel wird daraus ein 5,5 Kb-großes Fragment isoliert (Fig. 5).

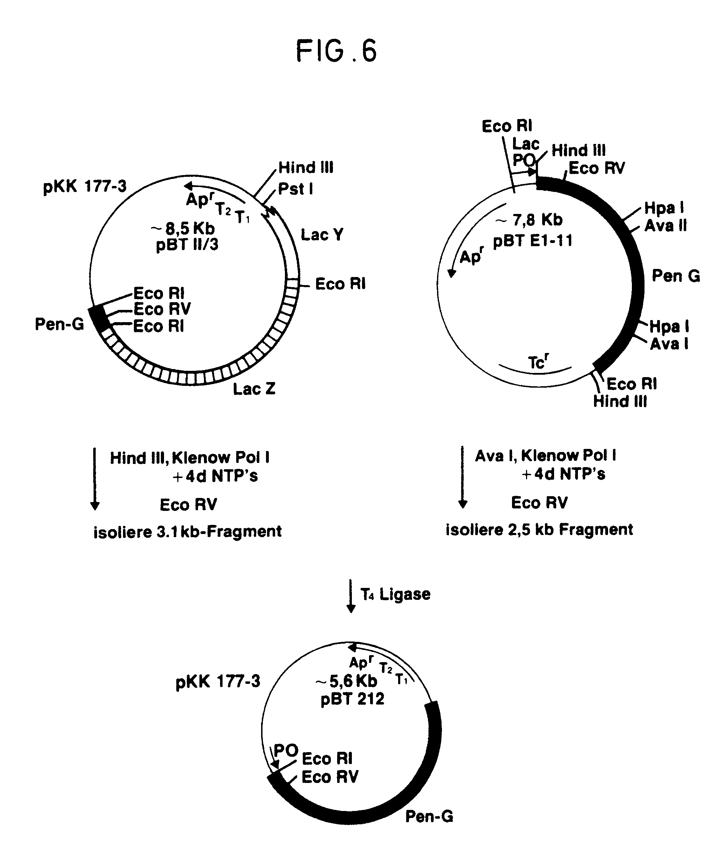

Ca. 100 ng des 2,9 Kb Vektor-Fragments aus pKK177-3 werden mit 200 ng des 5,5 Kb lac Z-Fragments aus pBT117 und 100 ng des 270 Bp-großen Eco RI-Fragments über Nacht mit 10 Einheiten T₄-Ligase ligiert (Fig. 5). Nach Transformation von E. coli 54-2 wurden β-Galaktosidaseposive Klone auf X-gal Indikatorplatten (Miller, J., H., (1972) in: Experiments in Molecular Genetics, Cold Spring Harbor Laboratory, 47-55) identifiziert. Durch Eco RI-Spaltung der Plasmid-DNA und Sequenzanalyse wird die gewünschte Konstruktion im Plasmid pBT II/3, DSM 3060 bestätigt (Fig. 5). Über die Eco RV-Spaltstelle kann die β-Galaktosidase-kodierende DNA entfernt und die Penicillin-Amidohydrolase-kodierende Region restauriert werden (Fig. 6). Plasmid pBT II/3 wird mit Hind III vollständig gespalten, die überstehenden Enden werden mit Polymerase I (Klenow-Fragment) und den 4-Desoxy-Ribonukleotid-Triphosphaten aufgefüllt, mit Eco RV wird vollständig gespalten und anschließend ein 3,1-Kb-Fragment nach Größenfraktionierung isoliert. Aus pBT E1-11 wird ein 2,5 Kb-Fragment über Eco RV- und Ava I-Spaltung isoliert. Vor der Eco RV-Spaltung wurden die überstehenden Ava I-Enden mit Polymerase I (Klenow-Fragment) und allen 4 Desoxy-Ribonukleotid-Triphosphaten gerade gemacht. Beide Fragmente (3,1 Kb und 2,5 Kb) werden in gleichen Mengenverhältnissen mit T₄-Ligase ligiert. Das resultierende Plasmid ist pBT212, DSM 3058. Dieses Plasmid kodiert eine Penicillin-G-Amidase ohne Signalsequenz (Fig. 6).

Aus Plasmid pBT212 wird durch Spaltung mit Eco RI und Hpa I ein ca. 720 Bp Fragment isoliert, aus diesem Fragment wird einerseits nach Spaltung mit Dde I ein ca. 400 Bp Eco RI, Dde I Fragment und andererseits nach Spaltung mit Alu I und Eco RV ein 410 Bp Fragment isoliert.

An das Eco RV, Alu I Fragment wird nach Denaturierung des Doppelstrangs ein 27mer-Primer mit der Sequenz

5' CCA AGC TTA TTA TGC TGT TTG CGA GTT 3'

hybridisiert.

Dieser nach der Phosphotriestermethode synthetisierte Primer (Crea et al., 1978. Proc. Natl. Acad. Sci, USA, 75, 5765-5769) ist homolog zum Gegenstrang von Position 691 bis 705, enthält das Stopkodon TAA 2-mal und eine Hind III-Erkennungssequenz.

Mit DNA-Polymerase (Klenow-Fragment) und den für die DNA-Synthese notwendigen Desoxytriphosphaten wird das nicht-hybridisierende 3'-Ende exonukleolytisch abgespalten und der Strang von 5' in Richtung 3' aufgefüllt. Die DNA wird mit Hind III und Dde I gespalten, und ein ca. 250 Bp Fragment isoliert. Das mit Eco RI und Hind III gespaltene Vektormolekül pKKI 77-3, das ca. 0.4 kb Eco RI, Dde I Fragment und das ca. 0,25 kb Dde I, Hind III Fragment werden mit Hilfe des Enzyms T4-Ligase verknüpft (siehe Fig. 9).

Das so gebildete Plasmid kodiert die kleine Untereinheit (alpha) der Penicillin-Amidohydrolase und trägt die Bezeichnung pBT 702, DSM 3067P.

## Beispiel 2

Der das Plasmid pBT 212 tragende E. coli-Stamm DSM 3058 wird über Nacht bei 37°C in Vollmedium in Gegenwart vom Induktor Isopropylthiogalaktosid (IPTG) angezogen. Die Zellen werden geerntet, aufgeschlossen und der Zellextrakt für 4 bis 8 Stunden bei 30°C inkubiert. Analyse der durch die Nachinkubation bei 30°C entstandenen Produkte im SDS-Acrylamid-Gel zeigt, daß eine Reifung des Vorläuferproteins in die α- und β-Untereinheit der Penicillin-G-Amidase stattfindet. Mit dem Auftreten der beiden Untereinheiten kann Enzymaktivität gemessen werden, d. h. proteolytische Spaltung und korrekte Faltung zum aktiven Enzym findet im Zellextrakt statt.

Durch Anreicherung der spezifischen Protease ist daher eine quantitative Spaltung des Vorläuferproteins zum aktiven Enzym möglich.

## Beispiel 3

Konstruktion eines Plasmids zur Expression der großen Untereinheit (β) der Penicillin-G-Amidase:

Zur Konstruktion eines neuen Startsignals (ATG) am Beginn der großen Untereinheit wurde die Technik einer Primer-gestarteten DNA-Synthese verwendet. Die große Untereinheit beginnt an Position 868 des sequenzierten Gens mit der Aminosäurefolge Ser, Asn, Met. Über die Phosphotriestermethode (Crea, R., Kaszewski, A., Hiros, T., Itakura, K., (1978) Proc. Natl. Acad. Sci., USA, 75, 5765-5769) wurde ein 25mer Primer mit der Basenfolge:

Met Ser Asn Met Trp Val
5'GGAATTC ATG AGC AAT ATG TGG GTT3'

synthetisiert.

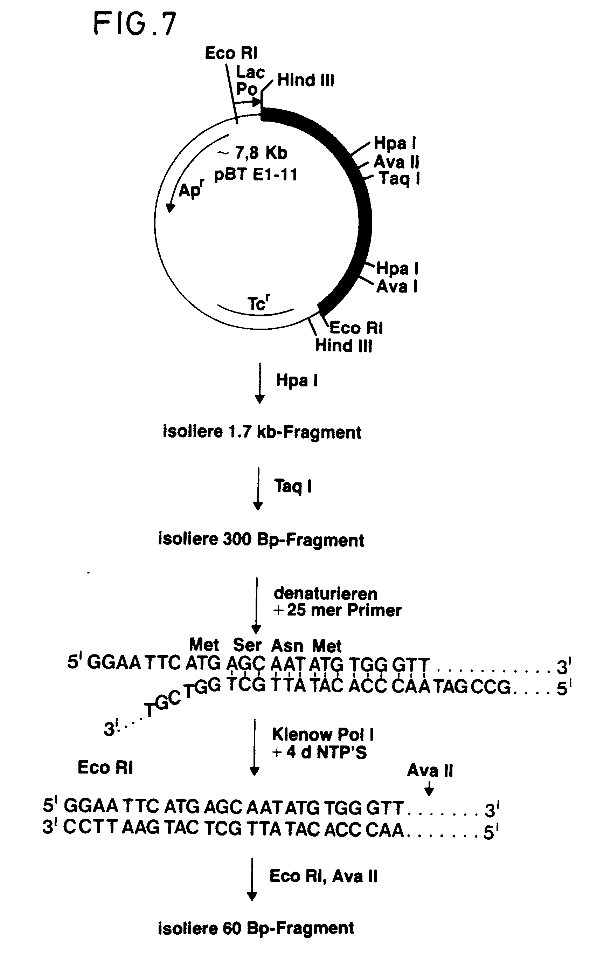

Dieser Primer enthält die Erkennungssequenz für die Restriktionsendonuklease Eco RI, ein ATG-Startkodon und die Basensequenz für die ersten 5 Aminosäuren der großen Untereinheit der Penicillin-G-Amidohydrolase (Fig. 7).

Aus Plasmid pBT E1-11 wurde durch Spaltung mit Hpa I und nach Größentrennung im Agarose-Gel ein 1,7 Kb-Fragment isoliert, dieses Fragment wird mit Taq I vollständig nachgespalten und ein 0,30 Kb-Fragment isoliert. Ca. 0,5 µg dieses Fragments werden durch Erhitzen für 5 Minuten bei 100°C denaturiert, 300 pMol des unbehandelten 25mer Primers werden zu dem Ansatz gegeben und auf Raumtemperatur abkühlen lassen. Nach Zugabe von 10 Einheiten DNA-Polymerase I (Klenow-Fragment) und allen 4 Desoxy-Ribonukleotid-Triphosphaten wird für 3 Stunden bei Raumtemperatur inkubiert und anschließend mit Ava II und Eco RI vollständig gespalten. Nach Größentrennung in einem 2,5%igen niedrigschmelzenden Agarose-Gel wird die Region, in der ein Fragment mit einer Größe von 60 Bp bandieren würde, ausgeschnitten, phenolysiert, die Probe ausgeethert und die DNA mit Ethanol gefällt.

Plasmid pKK177-3 wird mit Hind III und Eco RI vollständig gespalten. Durch Größenfraktionierung in einem 0,8%igem Agarose-Gel wird ein 2,9 Kb großes Hind III-Eco RI-Fragment isoliert.

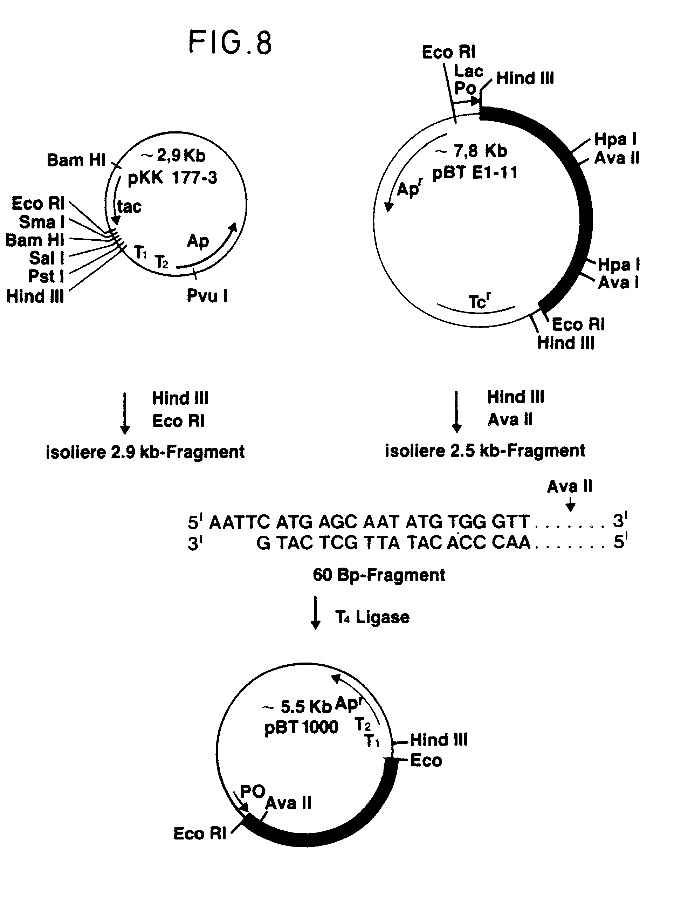

Plasmid pBT E1-11 wird mit Hind III und Ava II vollständig gespalten. Durch Größenfraktionierung in einem 0,8%igen Agarose-Gel wird ein 2,5 Kb Hind III-Ava II- Fragment isoliert (Fig. 8).

Je 0,1 µg dieser Fragmente werden zu dem Ethanol-gefällten 60 Bp-Eco RI-Ava II-Fragment gegeben. Nach Ligierung über Nacht wurde der E. coli-Stamm 54-2 transformiert, die Kolonien auf Schleicher/Schüll BA 85 Nitrozellulose-Filterpapier gestempelt und diese Filter auf LB Agarplatten, die 20 µg/ml Ampicillin enthielten, transferiert. Nach 6 Stunden Wachstum wurden die Filter auf LB-Agarplatten, die 20 µg/ml Ampicillin und 12,5 µg/ml Chlorampenicol enthielten, übertragen und über Nacht wachsen lassen. Die DNA der Kolonien wurde denaturiert und an Nitrozellulose-Filterpapier fixiert und anschließend mit dem radioaktiv-markierten 25mer Primer hybridisiert (mit Änderungen nach Davis, R., W., Botstein, D., Roth, J., R., (1980) in: Advanced Bacterial Genetics, Cold Spring Harbor Laboratory). Pro Filter wurden 10⁶ cpm für die Hybridisierung eingesetzt. Nach Waschung bei Raumtemperatur und bei 42°C wurden die getrockneten Filter für 3 Stunden bei Raumtemperatur mit einem Fuji RX Röntgenfilm exponiert. 15 Klone mit positivem Signal wurden identifiziert, über Eco RI-Spaltung die neukonstruierte Restriktionsstelle überprüft und die erwartete DNA- Sequenz durch Sequenzierung bestätigt. Das resultierende Plasmid trägt die Bezeichnung pBT1000, DSM 3068P (Fig. 8). Es codiert die große Untereinheit (β). Sie wurde nachgewiesen durch SDS-Gelchromatographie (64kD) und durch immunologische Identifizierung. Anfärbung mit Coomassie blue ergab, daß die β-Untereinheit ca. 30 bis 40 % des Gesamtproteins ausmacht.

**Beispiel 4**

Expression der alpha-Untereinheit der Penicillin-Amidohydrolase:
Der E. coli-Stamm K12 54-2, der Plasmid pBT 702 enthält, wird 12 Stunden bei 30°C in LB-Medium angezogen, anschließend 1:2 in Medium, das 2 mM IPTG enthält, verdünnt. Nach 4 Stunden Wachstum werden die Zellen geerntet, mit Ultraschall aufgeschlossen und die Bildung der alpha-Untereinheit auf SDS-Gel chromatographisch und immunologisch nachgewiesen. .

Dieses Ergebnis zeigt, daß von dem in pBT 702 enthaltenen inkompletten Gen die alpha-Untereinheit der Penicillin-G-Amidase kodiert wird.

**Beispiel 5**

Die nach den Beispielen 1 und 3 erhaltenen Plasmide wurden in die kompatiblen Plasmide pACYC 184 und pBR322 kloniert und gemeinsam in die Wirtszelle E. coli K12 54-2 transformiert. Benzyl-penicillin-spaltende Aktivität kann nach De- und Renaturierung der Zellextrakte nachgewiesen werden.

In den obigen Beispielen und in der Zeichnung stellen die angegebenen Fragmentgrößen ungefähre Angaben dar, die durch Vergleich mit Größenmarkern in Agarose-Gel erhalten wurden. Die genaue Nukleotidanzahl in den Fragmenten kann der Fachmann anhand der angegebenen DNA-Sequenz und der damit erkennbaren Restriktionsspaltstellen bestimmen.

**Ansprüche**

1. Zur Expression von Penicillin-G-Amidase geeignetes Plasmid, **dadurch gekennzeichnet,** daß es ein inkomplettes Penicillin-G-Amidase-Gen trägt, dem die ersten 78 Basen der translatierten Region am 5' Ende des kompletten Gens fehlen.

2. Plasmid nach Anspruch 1, **dadurch gekennzeichnet,** daß das inkomplette Penicillin-G-Amidase-Gen in Form von zwei getrennten Bruchstücken eingebaut vorliegt, die mit Base 79 bzw. 868 beginnen, gezählt vom Beginn der translatierten Region am 5' Ende des Gens und eine Deletion der kodierenden Basen 705 bis 867 aufweisen.

3. Plasmid nach Anspruch 1, **dadurch gekennzeichnet,** daß das translatierte inkomplette Penicillin-G-Amidase-Gen mit Base 79 beginnt und mit Base 705 endet.

4. Plasmid nach Anspruch 1, **dadurch gekennzeichnet,** daß das translatierte inkomplette Penicillin-G-Amidase-Gen mit Base 868 beginnt und nach Base 2538 endet, bzw. mit Base 2541 endet.

5. Plasmid nach einem der Ansprüche 1 bis 4, **dadurch gekennzeichnet,** daß mit dem 5'-Ende des inkompletten Penicillin-G-Amidase-Gen bzw. dessen Bruchstück die Basensequenz ATG verbunden ist.

6. Plasmid nach einem der vorhergehenden Ansprüche, **dadurch gekennzeichnet,** daß es zusätzlich ein Resistenzgen gegen Chloramphenicol, Tetracyclin oder/und Kanamyzin und einen bakteriellen Promotor enthält.

7. Plasmid nach Anspruch 6, **dadurch gekennzeichnet,** daß es den lac-Promotor oder den tac-Promotor unmittelbar vor dem Beginn des zu translatierenden neukonstruierten Genabschnittes enthält.

8. Plasmid pBT 702, DSM 3067P.

9. Plasmid pBT 1000, DSM 3068P.

10. Plasmid pBT 212 wie im Mikroorganismus DSM 3058 enthalten.

11. Mikroorganismus, **gekennzeichnet durch** einen Gehalt an wenigstens einem Plasmid nach einem der Ansprüche 1 bis 10.

12. Mikroorganismen nach Anspruch 11 mit der Hinterlegungsnummer DSM 3057 (pBT 1000), DSM 3064 (pBT 702) und DSM 3058 (pBT 212).

13. Verfahren zur Herstellung des Plasmids von Anspruch 1, **dadurch gekennzeichnet,** daß man Plasmid DSM 3060 (pBT II 3) mit Nuklease Hind III vollständig spaltet und die überstehenden Enden mit Polymerase I, Klenow-Fragment und dATP, dTTP, dGTP und dCTP auffüllt, danach mit Nuklease Eco RV vollständig spaltet und das gebildete 3.1 Kb-Fragment isoliert, Plasmid DSM 3061 (pBT E1-11) mit Nuklease Ava I spaltet, die überstehenden Enden mit Polymerase 1 Klenow-Fragment und dATP, dTTP, dGTP, dCTP auffüllt, danach mit Nuklease Eco RV spaltet und das gebildete 2,5 Kb-Fragment isoliert, das 3,1 Kb-Fragment und das 2,5 Kb-Fragment in gleichen Mengenverhältnissen mit T4-Ligase ligiert und Plasmid pBT 212 erhält.

14. Verfahren zur Herstellung eines Plasmids nach Anspruch 3, **dadurch gekennzeichnet,** daß man
   1) die DdeI-Erkennungssequenz an Position 77-81 des in einem Plasmid vorliegenden Pen-G-Amidase-Gens öffnet, mit Desoxythymidin- und Desoxycytosin-Triphosphat in Gegenwart von DNA-Polymerase die zurückstehende Sequenz auffällt, das am 5'-Ende überstehende Desoxythymidin mit der Nuklease S1 abspaltet und an die nun glatte Sequenz mit T4-Ligase einen Linker mit ATG-Startkodon und einer Restriktionsenzymerkennungssequenz anhängt,
   2) aus dem Penicillin-G-Amidase-Gen nach Spaltung mit Restriktionsendonukleasen Eco RV und Alu I ein 0,21 Kb-Fragment isoliert, denaturiert, dann mit einem Primer, der eine Erkennungssequenz für eine bestimmte Nuklease, daran gebunden ein oder mehrere Stopkodone, und an letztere die Basensequenz, die die Aminosäuren 235 bis 231 oder gegebenenfalls weniger oder mehr anschließende Aminosäuren kodiert, versetzt und in Gegenwart von Polymerase I (Klenow-Fragment) und den 4 für die DNA-Synthese notwendigen Desoxyribonukleotid-Triphosphaten inkubiert, die erhaltene DNA mit Dde I und der den Primer erkennenden Nuklease spaltet und das erhaltene Fragment von etwa 0,20 Kb mit einem geeigneten Fragment der Penicillin-G-Amidase ligiert, so daß
      a) eine Verknüpfung der durch Spaltung mit Dde I entstandenen überstehenden Enden an Position 455 der DNA-Sequenz und

b) eine Verknüpfung mit einem Expressionsvektor entsteht.

15. Verfahren nach Anspruch 14, **dadurch gekennzeichnet,** daß man Plasmid DSM 3058 (pBT 212) mit Eco RI und Dde I spaltet, ein 0,40 Kb-Fragment isoliert und einen Primer verwendet, der eine Hind III-Erkennungssequenz und 2 Stopkodone enthält, und daß man als Expressionsplasmid das mit Eco RI- und Hind III-gespaltene Plasmid DSM 3062 (pKK177-3) verwendet.

16. Verfahren zur Herstellung des Plasmids nach Anspruch 4, **dadurch gekennzeichnet,** daß man das Penicillin-G-Amidase-Gen mit Hpa I und Taq I spaltet, ein 300 Bp-Fragment isoliert und denaturiert, dann mit einem Primer, der eine Erkennungssequenz für eine bestimmte Nuklease, daran gebunden das Startkodon ATG und an letzteres die Basensequenz, welche die Aminosäuren 290 bis 294 und gegebenenfalls weitere oder aber auch weniger anschließende Aminosäuren codiert, versetzt und in Gegenwart von Polymerase I Klenow-Fragment und den 4 Desoxyribonukleotid-triphosphaten inkubiert, die erhaltene DNA mit Ava II und der den Primer erkennenden Nuklease spaltet und das erhaltene Fragment von etwa 60 Bp Länge
a) mit dem AvaII-HindIII-Fragment aus Plasmid DSM 3061 (pBT E1-11) und
b) mit einem durch Spaltung mit Hind III und der den Primer erkennenden Nuklease gebildeten Plasmidfragment ligiert.

17. Verfahren nach Anspruch 16, **dadurch gekennzeichnet,** daß man das Hind III-Eco R1-Fragment von Plasmid DSM 3062 (pKK 177-3) und einen Primer mit der Eco R1-Erkennungssequenz verwendet.

18. Verwendung eines Plasmids gemäß einem der Ansprüche 1 bis 10 zur Gewinnung von Penicillin-G-Amidase durch Expression des Enzyms in einen Mikroorganismus, der das Plasmid enthält.

19. Verwendung eines Plasmids gemäß Anspruch 18, **dadurch gekennzeichnet,** daß man ein Plasmid nach Anspruch 3 und ein Plasmid nach Anspruch 4 in einen gemeinsamen Wirts-Mikroorganismus exprimiert.

20. Verwendung eines Plasmids nach Anspruch 18, **dadurch gekennzeichnet,** daß man ein Plasmid nach Anspruch 3 in einen ersten Wirts-Mikroorganismus und ein Plasmid nach Anspruch 4 in einen zweiten Wirts-Mikroorganismus exprimiert und deren Extrakte vermischt.

21. Verwendung eines Plasmids gemäß Anspruch 18, **dadurch gekennzeichnet,** daß man ein Plasmid nach Anspruch 1 oder 2 in einen Wirts-Mikroorganismus exprimiert.

22. Verwendung eines Plasmids gemäß Anspruch 18 in E. coli 54-2, DSM 3066 oder DS 410, DSM 3065, DSM 3058 als Mikroorganismus.

Patentansprüche für folgenden Vertragsstaat: AT

1. Verfahren zur Herstellung von Penicillin-G-Amidase, **dadurch gekennzeichnet,** daß man ein Plasmid, welches ein inkomplettes Penicillin-G-Amidase-Gen trägt, dem die ersten 78 Basen der translatierten Region am 5' Ende des kompletten Gens fehlen, in einen Mikroorganismus, der das Plasmid enthält, exprimiert.

2. Verfahren nach Anspruch 1, **dadurch gekennzeichnet,** daß man ein Plasmid verwendet, welches das inkomplette Penicillin-G-Amidase-Gen in Form von zwei getrennten Bruchstücken eingebaut enthält, die mit Base 79 bzw. 868 beginnen, gezählt vom Beginn der translatierten Region am 5' Ende des Gens und eine Deletion der kodierenden Basen 705 bis 867 aufweisen.

3. Verfahren nach Anspruch 1, **dadurch gekennzeichnet,** daß man ein Plasmid verwendet, in dem das translatierte inkomplette Penicillin-G-Amidase-Gen mit Base 79 beginnt und mit Base 705 endet.

4. Verfahren nach Anspruch 1, **dadurch gekennzeichnet,** daß man ein Plasmid verwendet, in dem das translatierte inkomplette Penicillin-G-Amidase-Gen mit Base 868 beginntund nach Base 2538 endet bzw. mit Base 2541 endet.

5. Verfahren nach einem der Ansprüche 1 bis 4, **dadurch gekennzeichnet,** daß man ein Plasmid verwendet, in dem mit dem 5' Ende des inkompletten Penicillin-G-Amidase-Gens bzw. dessen Bruchstücks die Basensequenz ATG verbunden ist.

6. Verfahren nach einem der vorhergehenden Ansprüche,
**dadurch gekennzeichnet,**
daß das Plasmid zusätzlich ein Resistenzgen gegen Chloramphenicol, Tetracyclin oder/und Kanamyzin und einen bakteriellen Promotor enthält.

7. Verfahren nach Anspruch 6,
**dadurch gekennzeichnet,**
daß das Plasmid den lac-Promotor oder den tac-Promotor unmittelbar vor dem Beginn des zu translatierenden neukonstruierten Genabschnittes enthält.

8. Verfahren nach Anspruch 1,
**dadurch gekennzeichnet,**
daß man Plasmid pBT 702, DSM 3067P, Plasmid pBT 1000, DSM 3068P oder Plasmid pBT 212 wie im Mikroorganismus DSM 3058 enthalten, verwendet.

9. Verfahren zur Herstellung eines Plasmids für das Verfahren von Anspruch 1,
**dadurch gekennzeichnet,**
daß man Plasmid DSM 3060 (pBT II/3) mit Nuklease Hind III vollständig spaltet und die überstehenden Enden mit Polymerase I, Klenow-Fragment und dATP, dTTP, dGTP und dCTP auffüllt, danach mit Nuklease Eco PV vollständig spaltet und das gebildete 3,1 Kb-Fragment isoliert, Plasmid DSM 3061 (pBT E1-11) mit Nuklease Ava I spaltet, die überstehenden Enden mit Polymerase 1 Klenow-Fragment und dATP, dTTP, dGTP, dCTP auffüllt, danach mit Nuklease Eco PV spaltet und das gebildete 2,5 Kb-Fragment isoliert, das 3,1 Kb-Fragment und das 2,5 Kb-Fragment in gleichen Mengenverhältnissen mit T4-Ligase ligiert und Plasmid pBT 212 erhält.

10. Verfahren zur Herstellung eines Plasmids für das Verfahren nach Anspruch 3,
**dadurch gekennzeichnet,**
daß man
1) die DdeI-Erkennungssequenz an Position 77-81 des in einem Plasmid vorliegenden Pen-G-Amidase-Gens öffnet, mit Desoxythymidin- und Desoxycytosin-Triphosphat in Gegenwart von DNA-Polymerase die zurückstehende Sequenz auffällt, das am 5'-Ende überstehende Desoxythymidin mit der Nuklease S1 abspaltet und an die nun glatte Sequenz mit T4-Ligase einen Linker mit ATG-Startkodon und einer Restriktionsenzymerkennungssequenz anhängt.
2) aus dem Penicillin-G-Amidase-Gen nach Spaltung mit Restriktionsendonukleasen Eco RV und Alu I ein 0,21 Kb-Fragment isoliert, denaturiert, dann mit einem Primer, der eine Erkennungssequenz für eine bestimmte Nuklease, daran gebunden ein oder mehrere Stopkodone, und an letztere die Basensequenz, die die Aminosäuren 235 bis 231 oder gegebenenfalls weniger oder mehr anschließende Aminosäuren kodiert, versetzt und in Gegenwart von Polymerase I (Klenow-Fragment) und den 4 für die DNA-Synthese notwendigen Desoxyribonukleotid-Triphosphaten inkubiert, die erhaltene DNA mit Dde I und der den Primer erkennenden Nuklease spaltet und das erhaltene Fragment von etwa 0,20 Kb mit einem geeigneten Fragment der Penicillin-G-Amidase ligiert, so daß
a) eine Verknüpfung der durch Spaltung mit Dde I entstandenen überstehenden Enden an Position 455 der DNA-Sequenz und
b) eine Verknüpfung mit einem Expressionsvektor entsteht.

11. Verfahren nach Anspruch 10,
**dadurch gekennzeichnet,**
daß man Plasmid DSM 3058 (pBT 212) mit Eco RI und Dde I spaltet, ein 0,40 Kb-Fragment isoliert und einen Primer verwendet, der eine Hind III-Erkennungssequenz und 2 Stopkodone enthält, und daß man als Expressionsplasmid das mit Eco RI- und Hind III-gespaltene Plasmid DSM 3062 (pKK177-3) verwendet.

12. Verfahren zur Herstellung eines Plasmids für das Verfahren nach Anspruch 4,
**dadurch gekennzeichnet,**
daß man das Penicillin-G-Amidase-Gen mit Hpa I und Taq I spaltet, ein 300 Bp-Fragment isoliert und denaturiert, dann mit einem Primer, der eine Erkennungssequenz für eine bestimmte Nuklease, daran gebunden das Startkodon ATG und an letzteres die Basensequenz, welche die Aminosäuren 290 bis 294 und gegebenenfalls weitere oder aber auch weniger anschließende Aminosäuren codiert, versetzt und in Gegenwart von Polymerase I Klenow-Fragment und den 4 Desoxyribonukleotid-triphosphaten inkubiert, die erhaltene DNA mit Ava II und der den Primer erkennenden Nuklease spaltet und das erhaltene Fragment von etwa 60 Bp Länge
a) mit dem AvaII-HindIII-Fragment aus Plasmid DSM 3061 (pBT E1-11) und
b) mit einem durch Spaltung mit Hind III und der den Primer erkennenden Nuklease gebildeten Plasmidfragment ligiert.

13. Verfahren nach Anspruch 12,
**dadurch gekennzeichnet,**
daß man das Hind III-Eco R1-Fragment von Plasmid DSM 3062 (pKK 177-3) und einen Primer mit der Eco R1-Erkennungssequenzverwendet.

14. Verfahren gemäß Anspruch 1,
**dadurch gekennzeichnet,**
daß man ein Plasmid nach Anspruch 3 und ein Plasmid nach Anspruch 4 in einen gemeinsamen Wirts-Mikroorganismus exprimiert.

15. Verfahren nach Anspruch 1,
**dadurch gekennzeichnet,**
daß man ein Plasmid nach Anspruch 3 in einen ersten Wirts-Mikroorganismus und ein Plasmid nach Anspruch 4 in einen zweiten Wirts-Mikroorganismus exprimiert und deren Extrakte vermischt.

16. Verfahren gemäß Anspruch 1,
**dadurch gekennzeichnet,**
daß man das Plasmid in E.coli 54-2, DSM 3066 oder DS 410, DSM 3065, DSM 3058 als Mikroorganismus exprimiert.

## Claims

1. Plasmid suitable for the expression of penicillin G amidase, characterised in that it carries an incomplete penicillin G amidase gene in which the first 78 bases of the translation region on the 5' end of the complete gene are missing.

2. Plasmid according to claim 1, characterised in that the incomplete penicillin G amidase gene is present incorporated in the form of two separate fragments which commence with bases 79 and 868, respectively, counting from the beginning of the translated region on the 5' end of the gene and displaying a deletion of the coding bases 705 to 867.

3. Plasmid according to claim 1, characterised in that the translated incomplete penicillin G amidase gene begins with base 79 and ends with base 705.

4. Plasmid according to claim 1, characterised in that the translated incomplete penicillin G amidase gene begins with base 868 and ends after base 2538 or with base 2541.

5. Plasmid according to one of claims 1 to 4, characterised in that the base sequence ATG is connected with the 5' end of the incomplete penicillin G amidase gene or its fragment.

6. Plasmid according to one of the preceding claims, characterised in that it additionally contains a resistance gene against chloramphenicol, tetracycline and/or kanamycin and a bacterial promotor.

7. Plasmid according to claim 6, characterised in that it contains the lac promotor or the tac promotor immediately before the beginning of the newly constructed gene section to be translated.

8. Plasmid pBT 702, DSM 3067P.

9. Plasmid pBT 1000, DSM 3068P.

10. Plasmid pBT 212 as contained in the micro-organism DSM 3058.

11. Micro-organism characterised by a content of at least one plasmid according to one of claims 1 to 10.

12. Micro-organisms according to claim 11 with the deposit numbers DSM 3057 (pBT 1000), DSM 3064 (pBT 702) and DSM 3058 (pBT 212).

13. Process for the production of the plasmid of claim 1, characterised in that one completely cleaves plasmid DSM 3060 (pBT II/3) with nuclease Hind III and fills the projecting ends with polymerase I, Klenow fragment and dATP, dTTP, dGTP and dCTP, thereafter completely cleaves with nuclease Eco RV and isolates the 3.1 Kb fragment formed, cleaves plasmid DSM 3061 (pBT E1-11) with nuclease Ava I, fills the projecting ends with polymerase I, Klenow fragment and dATP, dTTP, dCTP, dCTP, thereafter cleaves with nuclease Eco PV and isolates the 2.5 Kb fragment formed, ligates the 3.1 Kb fragment and the 2.5 Kb fragment with T₄ ligase in equal amount ratios and obtains plasmid pBT 212.

14. Process for the production of a plasmid according to claim 3, characterised in that one
1) opens the Dde I recognition sequence at position 77-81 of the pen. G amidase gene present in a plasmid, fills the remaining sequence with desoxythymidine and desoxycytosine triphosphate in the presence of DNA polymerase, splits off the desoxythymidine remaining on the 5' end with nuclease S1 and attaches a linker with ATG start codon and a restriction enzyme recognition sequence with $T_4$ ligase to the now smooth sequence,

2) isolates a 0.21 Kb fragment from the penicillin G amidase gene after cleavage with restriction endonucleases Eco RV and Alu I, denatures, then mixes with a primer, which codes a recognition sequence for a particular nuclease, one or more stop codons attached thereto, and, on the latter, the base sequence which codes the amino acids 235 to 231 or optionally less or more following amino acids, and incubates in the presence of polymerase I (Klenow fragment) and the 4 desoxyribonucleotide triphosphates necessary for the DNA synthesis, cleaves the DNA obtained with Dde I and the nuclease recognising the primer and ligates the fragment obtained of about 0.20 Kb with a suitable fragment of the penicillin G amidase so that

    a) a linking of the projecting ends resulting by cleavage with Dde I to position 455 of the DNA sequence and
    b) a linking with an expression vector results.

15. Process according to claim 14, characterised in that one cleaves plasmid DSM 3058 (pBT 212) with Eco RI and Dde I, isolates a 0.40 Kb fragment and uses a primer which contains a Hind III recognition sequence and 2 stop codons and that, as expression plasmid, one uses plasmid DSM 3062 (pKK 177-3) cleaved with Eco RI and Hind III.

16. Process for the production of the plasmid according to claim 4, characterised in that one cleaves the penicillin G amidase gene with Hpa I and Taq I, isolates a 300 Bp fragment and denatures, then mixes with a primer, which codes a recognition sequence for a particular nuclease, to which is bound the start codon ATG and on the latter the base sequence which codes the amino acids 290 to 294 and possibly further or, however, also less following amino acids, and incubates in the presence of polymerase I Klenow fragment and the 4 desoxyribonucleotide triphosphates, cleaves the DNA obtained with Ava II and the nuclease recognising the primer and ligates the fragment obtained of about 60 Bp length

    a) with the Ava II-Hind III fragment from plasmid DSM 3061 (pBT E1-11) and
    b) with a plasmid fragment formed by cleavage with Hind III and the nuclease recognising the primer.

17. Process according to claim 16, characterised in that one uses the Hind III - Eco RI fragment of plasmid DSM 3062 (pKK 177-3) and a primer with the Eco RI recognition sequence.

18. Use of a plasmid according to one of claims 1 to 10 for the obtaining of penicillin G amidase by expression of the enzyme in a micro-organism which contains the plasmid.

19. Use of a plasmid according to claim 18, characterised in that one expresses a plasmid according to claim 3 and a plasmid according to claim 4 in a common host micro-organism.

20. Use of a plasmid according to claim 18, characterised in that one expresses a plasmid according to claim 3 in a first host micro-organism and a plasmid according to claim 4 in a second host micro-organism and mixes their extracts.

21. Use of a plasmid according to claim 18, characterised in that one expresses a plasmid according to claim 1 or 2 in a host micro-organism.

22. The use of a plasmid according to claim 18 in E. coli 54-2, DSM 3066 or DS 410, DSM 3065, DSM 3058 as micro-organism.

Claims for the following Contracting State: AT

1. Process for the preparation of penicillin G amidase, characterised in that one expresses a plasmid which carries an incomplete penicillin G amidase gene, in which the first 78 bases of the translation region on the 5' end of the complete gene are missing, in a micro-organism which contains the plasmid.

2. Process according to claim 1, characterised in that one uses a plasmid which contains incorporated the incomplete penicillin G amidase gene present in the form of two separate fragments which commence with bases 79 and 868, respectively, counting from the beginning of the translated region on the 5' end of the gene and display a deletion of the coding bases 705 to 867.

3. Process according to claim 1, characterised in that one uses a plasmid in which the translated incomplete penicillin G amidase gene begins with base 79 and ends with base 705.

4. Process according to claim 1, characterised in that one uses a plasmid in which the translated incomplete penicillin G amidase gene begins with base 868 and ends after base 2538 or with base 2541.

5. Process according to one of claims 1 to 4, characterised in that one uses a plasmid in which the base sequence ATG is connected with the 5' end of the incomplete penicillin G amidase gene or with its fragment.

6. Process according to one of the preceding claims, characterised in that the plasmid additionally contains a resistance gene against chloramphenicol, tetracycline and/or kanamycin and a bacterial promotor.

7. Process according to claim 6, characterised in that the plasmid contains the 1ac promotor or the tac promotor immediately before the beginning of the newly constructed gene section to be translated.

8. Process according to claim 1, characterised in that one uses plasmid pBT 702, DSM 3067P, plasmid pBT 1000, DSM 3068P or plasmid pBT 212 as contained in micro-organism DSM 3058.

9. Process for the production of a plasmid for the process of claim 1, characterised in that one completely cleaves plasmid DSM 3060 (pBT II/3) with nuclease Hind III and fills the projecting ends with polymerase I, Klenow fragment and dATP, dTTP, dGTP and dCTP, thereafter completely cleaves with nuclease Eco RV and isolates the 3.1 Kb fragment formed, cleaves plasmid DSM 3061 (pBT E1-11) with nuclease Ava I, fills the projecting ends with polymerase I, Klenow fragment and dATP, dTTP, dGTP, dCTP, thereafter cleaves with nuclease Eco PV and isolates the 2.5 Kb fragment formed, ligates the 3.1 Kb fragment and the 2.5 Kb fragment with $T_4$ ligase in equal amount ratios and obtains plasmid pBT 212.

10. Process for the production of a plasmid for the process according to claim 3, characterised in that one
    1) opens the Dde I recognition sequence at position 77-81 of the pen. G amidase gene present in a plasmid, fills the remaining sequence with desoxythymidine and desoxycytosine triphosphate in the presence of DNA polymerase, splits off the desoxythymidine remaining on the 5' end with nuclease S1 and attaches a linker with ATG start codon and a restriction enzyme recognition sequence with $T_4$ ligase to the now smooth sequence,
    2) isolates a 0.21 Kb fragment from the penicillin G amidase gene after cleavage with the restriction endonucleases Eco RV and Alu I, denatures, then mixes with a primer, which codes a recognition sequence for a particular nuclease, one or more stop codons attached thereto, and, on the latter, the base sequence which codes the amino acids 235 to 231 or optionally less or more following amino acids, and incubates in the presence of polymerase I (Klenow fragment) and the 4 desoxyribonucleotide triphosphates necessary for the DNA synthesis, cleaves the DNA obtained with Dde I and the nuclease recognising the primer and ligates the fragment obtained of about 0.20 Kb with a suitable fragment of the penicillin G amidase so that
      a) a linking of the projecting ends resulting by cleavage with Dde I to position 455 of the DNA sequence and
      b) a linking with an expression vector results.

11. Process according to claim 10, characterised in that one cleaves plasmid DSM 3058 (pBT 212) with Eco RI and Dde I, isolates a 0.40 Kb fragment and uses a primer which contains a Hind III recognition sequence and 2 stop codons and that, as expression plasmid, one uses plasmid DSM 3062 (pKK 177-3) cleaved with Eco RI and Hind III.

12. Process for the production of a plasmid for the process according to claim 4, characterised in that one cleaves the penicillin G amidase gene with Hpa I and Taq I, isolates a 300 Bp fragment and denatures, then mixes with a primer, which codes a recognition sequence for a particular nuclease, to which is bound the start codon ATG and on the latter the base sequence which codes the amino acids 290 to 294 and possibly further or, however, also less following amino acids, and incubates in the presence of polymerase I Klenow fragment and the 4 desoxyribonucleotide triphosphates, cleaves the DNA obtained with Ava II and the nuclease recognising the primer and ligates the fragment obtained of about 60 Bp length
      a) with the Ava II-Hind III fragment from plasmid pBT E1-11 and
      b) with a plasmid fragment formed by cleavage with Hind III and the nuclease recognising the primer.

13. Process according to claim 12, characterised in that one uses the Hind III - Eco RI fragment of plasmid DSM 3062 (pKK 177-3) and a primer with the Eco RI recognition sequence.

14. Process according to claim 1, characterised in that one expresses a plasmid according to claim 3 and a plasmid according to claim 4 in a common host micro-organism.

15. Process according to claim 1, characterised in that one expresses a plasmid according to claim 3 in a first host micro-organism and a plasmid according to claim 4 in a second host micro-organism, mixes their extracts.

16. Process according to claim 1, characterised in that one expresses the plasmid in E. coli 54-2, DSM 3066 or DS 410, DSM 3065, DSM 3058 as micro-organism.

## Revendications

1. Plasmide convenant à l'expression de la pénicilline-G-amidase, caractérisé en ce qu'il porte un gène incomplet de la pénicilline-G-amidase auquel manquent les 78 premières bases de la région traduite à l'extrémité 5' du gène complet.

2. Plasmide selon la revendication 1, caractérisé en ce que le gène incomplet de la pénicilline-G-amidase est présent sous forme de deux fragments séparés qui commencent par la base 79 et 868, comptées à partir du début de la région traduite à l'extrémité 5' du gène et une délétion des bases codantes 705 à 867.

3. Plasmide selon la revendication 1, caractérisé en ce que le gène incomplet de la pénicilline-G-amidase traduit commence par la base 79 et se termine par la base 705.

4. Plasmide selon la revendication 1, caractérisé en ce que le gène incomplet de la pénicilline-G-amidase traduit commence par la base 868 et se termine après la base 2538 ou par la base 2541.

5. Plasmide selon l'une des revendications 1 à 4, caractérisé en ce que la séquence de bases ATG est liée à l'extrémité 5' du gène incomplet de la pénicilline-G-amidase ou de son fragment.

6. Plasmide selon l'une des revendications précédentes, caractérisé en ce qu'il contient en outre un gène de résistance contre le chloramphénicol, la tétracycline et/ou la kanamycine et un promoteur bactérien.

7. Plasmide selon la revendication 6. caractérisé en ce qu'il contient le promoteur lac ou le promoteur tac immédiatement avant le début du segment de gène reconstruit qui doit être traduit.

8. Plasmide pBT 702, DSM 3067P.

9. Plasmide pBT 1000, DSM 3068P

10. Plasmide pBT 212, tel que contenu dans le micro-organisme DSM 3058.

11. Micro-organisme, caractérisé par une teneur en au moins un plasmide selon l'une des revendications 1 à 10.

12. Micro-organismes selon la revendication 11 portant les numéros de dépôt DSM 3057 (pBT 1000), DSM 3064 (pBT 702) et DSM 3058 (pBT 212).

13. Procédé d'obtention du plasmide selon la revendication 1, caractérisé en ce que l'on coupe totalement le plasmide DSM 3060 (pBT II 3) avec la nucléase Hind III et l'on remplit les extrémités portant des prolongements simple brin avec la polymérase I, fragment de Klenow, et dATP, dTTP, dGTP et dCTP, puis on coupe totalement avec la nucléase Eco PV et on isole le fragment de 3,1 kb formé. on coupe le plasmide DSM 3061 (pBT E1-11) avec la nucléase Ava I, on remplit les extrémités portant des prolongements simple brin avec la polymérase I, fragment de Klenow. et dATP. dTTP. dGTP. dCTP. puis on coupe avec la nucléase Eco PV et on isole le fragment de 2,5 kb formé. on ligature le fragment de 3.1 kb et le fragment de 2.5 kb suivant les mêmes rapports pondéraux avec la ligase T₄ et on obtient le plasmide pBT 212.

14. procédé d'obtention d'un plasmide selon la revendication 3. caractérisé en ce que l'on
   1) ouvre la séquence de reconnaissance Ddel en position 77-81 du gène de la pénicilline-G-amidase présent dans un plasmide. on remplit la séquence en retrait avec du desoxythymidine- et desoxycytosine-phosphate en présence d'ADN-polymérase, on sépare la desoxythymidine qui dépasse à l'extrémité 5' avec la nucléase S1 et, avec la ligase T₄. on ajoute à la séquence maintenant franche un lieur avec un codon d'initiation ATG et une séquence de reconnaissance d'enzyme de restriction,
   2) à partir du gène de la pénicilline-G-amidase, après coupure avec les endonucléases de restriction Eco RV et Alu I, on isole un fragment de 0,21 kb, on le dénature, puis on l'additionne d'une amorce qui contient une séquence de reconnaissance pour une nucléase déterminée, un ou plusieurs codons d'arrêt qui y sont liés et la séquence de bases liée à ceux-ci qui code pour les acides aminés 235 à 231 ou éventuellement moins ou plus d'acides aminés suivants, et on incube en présence de polymérase I (fragment de Klenow) et des quatre desoxyribonucléotide-triphosphates nécessaires pour la synthèse d'ADN, on coupe l'ADN obtenu avec Dde I et la nucléase qui reconnait l'amorce et on ligature le fragment obtenu d'environ 0,20 kb avec un fragment approprié de la pénicilline-G-amidase, de sorte qu'il en résulte

a) une liaison des extrémités présentant des prolongements simple brin formées par coupure par Dde I en position 455 de la séquence d'ADN et

b) une liaison avec un vecteur d'expression.

15. Procédé selon la revendication 14, caractérisé en ce que l'on coupe un plasmide DSM 3058 (pBT 212) avec Eco RI et Dde I, on isole un fragment de 0,40 kb et on utilise une amorce qui contient une séquence de reconnaissance de Hind III et deux codons d'arrêt, et en ce que l'on utilise comme plasmide d'expression le plasmide DSM 3062 (pKK177-3) coupé par Eco RI et Hind III.

16. Procédé d'obtention du plasmide selon la revendication 4, caractérisé en ce que l'on coupe le gène de la pénicilline-G-amidase avec Hpa I et Taq I, on isole un fragment de 300 pb et on le dénature, puis on additionne d'une amorce qui contient une séquence de reconnaissance pour une nucléase déterminée, le codon d'initiation ATG qui y est lié et la séquence de bases liée à celui-ci qui code pour les acides aminés 290 à 294 et éventuellement plus ou moins d'autres acides aminés suivants, et on incube en présence de polymérase I (fragment de Klenow) et des 4 désoxyribonucléotide-phosphates, on coupe l'ADN obtenu avec Ava II et la nucléase qui reconnait l'amorce et on ligature le fragment obtenu d'une longueur d'environ 60 pb

a) avec le fragment Ava II-Hind III du plasmide DSM 3061 (pBT E1-11) et

b) avec un fragment de plasmide formé par coupure avec Hind III et la nucléase qui reconnait l'amorce.

17. Procédé selon la revendication 16, caractérisé en ce que l'on utilise le fragment Hind III-Eco RI du plasmide DSM 3062 (pKK 177-3) et une amorce avec la séquence de reconnaissance de Eco RI.

18. Utilisation d'un plasmide selon l'une des revendications 1 à 10 pour l'obtention de pénicilline-G-amidase par expression de l'enzyme dans un micro-organisme qui contient le plasmide.

19. Utilisation d'un plasmide selon la revendication 18, caractérisée en ce que l'on exprime un plasmide selon la revendication 3 et un plasmide selon la revendication 4 dans un micro-organisme hôte commun.

20. Utilisation d'un plasmide selon la revendication 18, caractérisée en ce que l'on exprime un plasmide selon la revendication 3 dans un premier micro-organisme hôte et un plasmide selon la revendication 4 dans un second micro-organisme hôte et on mélange leurs extraits.

21. Utilisation d'un plasmide selon la revendication 18, caractérisée en ce que l'on exprime un plasmide selon la revendication 1 ou 2 dans un micro-organisme hôte.

22. Utilisation d'un plasmide selon la revendication 18 dans E.coli 54-2, DSM 3066 ou DS 410, DSM 3065, DSM 3058 en tant que micro-organisme.

Revendications pour L'Etat contractant suivant: AT

1. Procédé de préparation de la pénicilline-G-amidase, caractérisé en ce que l'on exprime un plasmide qui porte un gène incomplet de la pénicilline-G-amidase auquel manquent les 78 premières bases de la région traduite à l'extrémité 5' du gène complet dans un micro-organisme qui contient le plasmide.

2. Procédé selon la revendication 1, caractérisé en ce que l'on utilise un plasmide qui contient le gène incomplet de la pénicilline-G-amidase présent sous forme de deux fragments séparés qui commencent par la base 79 et 868, comptées à partir du début de la région traduite à l'extrémité 5' du gène et une délétion des bases codantes 705 à 867.

3. Procédé selon la revendication 1, caractérisé en ce que l'on utilise un plasmide dans lequel le gène incomplet de la pénicilline-G-amidase traduit commence par la base 79 et se termine par la base 705.

4. Procédé selon la revendication 1, caractérisé en ce que l'on utilise un plasmide dans lequel le gène incomplet de la pénicilline-G-amidase traduit commence par la base 868 et se termine après la base 2538 ou par la base 2541.

5. Procédé selon l'une des revendications 1 à 4, caractérisé en ce que l'on utilise un plasmide dans lequel la séquence de bases ATG est liée à l'extrémité 5' du gène incomplet de la pénicilline-G-amidase ou de son fragment.

6. Procédé selon l'une des revendications précédentes, caractérisé en ce que le plasmide contient en outre un gène de résistance contre le chloramphénicol, la tétracycline et/ou la kanamycine et un promoteur bactérien.

7. Procédé selon la revendication 6, caractérisé en ce que le plasmide contient le promoteur lac ou le promoteur tac immédiatement avant le début du segment de gène reconstruit qui doit être traduit.

8. Procédé selon la revendication 1, caractérisé en ce que l'on utilise le plasmide pBT 702, DSM 3067P, le plasmide pBT 1000, DSM 3068P ou le plasmide pBT 212, tel que contenu dans le micro-organisme DSM 3058.

9. Procédé d'obtention d'un plasmide pour le procédé selon la revendication 1, caractérisé en ce que l'on coupe totalement le plasmide DSM 3060 (pBT II/3) avec la nucléase Hind III et l'on remplit les extrémités portant des prolongements simple brin avec la polymérase I, fragment de Klenow, et dATP, dTTP, dGTP et dCTP, puis on coupe totalement avec la nucléase Eco PV et on isole le fragment de 3,1 kb formé, on coupe le plasmide DSM 3061 (pBT E1-11) avec la nucléase Ava I, on remplit les extrémités portant des prolongements simple brin avec la polymérase I, fragment de Klenow, et dATP, dTTP, dGTP, dCTP, puis on coupe avec la nucléase Eco PV et on isole le fragment de 2,5 kb formé, on ligature le fragment de 3,1 kb et le fragment de 2,5 kb suivant les mêmes rapports pondéraux avec la ligase T₄ et on obtient le plasmide pBT 212.

10. Procédé d'obtention d'un plasmide pour le procédé selon la revendication 3, caractérisé en ce que l'on
   1) ouvre la séquence de reconnaissance DdeI en position 77-81 du gène de la pénicilline-G-amidase présent dans un plasmide, on remplit la séquence en retrait avec du desoxythymidine- et desoxycytosine-phosphate en présence d'ADN-polymérase, on sépare la desoxythymidine qui dépasse à l'extrémité 5' avec la nucléase S1 et, avec la ligase T₄, on ajoute à la séquence maintenant franche un lieur avec un codon d'initiation ATG et une séquence de reconnaissance d'enzyme de restriction,
   2) à partir du gène de la pénicilline-G-amidase, après coupure avec les endonucléases de restriction Eco RV et Alu I, on isole un fragment de 0,21 kb, on le dénature, puis on l'additionne d'une amorce qui contient une séquence de reconnaissance pour une nucléase déterminée, un ou plusieurs codons d'arrêt qui y sont liés et la séquence de bases liée à ceux-ci qui code pour les acides aminés 235 à 231 ou éventuellement moins ou plus d'acides aminés suivants, et on incube en présence de polymérase I (fragment de Klenow) et des quatre desoxyribonucléotide-triphosphates nécessaires pour la synthèse d'ADN, on coupe l'ADN obtenu avec Dde I et la nucléase qui reconnait l'amorce et on ligature le fragment obtenu d'environ 0,20 kb avec un fragment approprié de la pénicilline-G-amidase, de sorte qu'il en résulte
       a) une liaison des extrémités présentant des prolongements simple brin formées par coupure par Dde I en position 455 de la séquence d'ADN et
       b) une liaison avec un vecteur d'expression.

11. Procédé selon la revendication 10, caractérisé en ce que l'on coupe un plasmide DSM 3058 (pBT 212) avec Eco RI et Dde I, on isole un fragment de 0,40 kb et on utilise une amorce qui contient une séquence de reconnaissance de Hind III et deux codons d'arrêt, et en ce que l'on utilise comme plasmide d'expression le plasmide DSM 3062 (pKK177-3) coupé par Eco RI et Hind III.

12. Procédé d'obtention d'un plasmide pour le procédé selon la revendication 4, caractérisé en ce que l'on coupe le gène de la pénicilline-G-amidase avec Hpa I et Taq I, on isole un fragment de 300 pb et on le dénature, puis on additionne d'une amorce qui contient une séquence de reconnaissance pour une nucléase déterminée, le codon d'initiation ATG qui y est lié et la séquence de bases liée à celui-ci qui code pour les acides aminés 290 à 294 et éventuellement plus ou moins d'autres acides aminés suivants, et on incube en présence de polymérase I (fragment de Klenow) et des 4 désoxyribonucléotide-phosphates, on coupe l'ADN obtenu avec Ava II et la nucléase qui reconnait l'amorce et on ligature le fragment obtenu d'une longueur d'environ 60 pb

    a) avec le fragment Ava II-Hind III du plasmide DSM 3061 (pBT E1-11) et

    b) avec un fragment de plasmide formé par coupure avec Hind III et la nucléase qui reconnait l'amorce.

13. Procédé selon la revendication 12, caractérisé en ce que l'on utilise le fragment Hind III-Eco RI du plasmide DSM 3062 (pKK 177-3) et une amorce avec la séquence de reconnaissance de Eco RI.

14. Procédé selon la revendication 1, caractérisé en ce que l'on exprime un plasmide selon la revendication 3 et un plasmide selon la revendication 4 dans un micro-organisme hôte commun.

15. Procédé selon la revendication 1, caractérisé en ce que l'on exprime un plasmide selon la revendication 3 dans un premier micro-organisme hôte et un plasmide selon la revendication 4 dans un second micro-organisme hôte et on mélange leurs extraits.

16. Procédé selon la revendication 1, caractérisé en ce que l'on exprime le plasmide dans E. coli 54-2, DSM 3066 ou DS 410, DSM 3065, DSM 3058 en tant que micro-organisme.

# FIG.1

AAGCTTCGTTGCTAGTATCAATTCGCTAATTATACACCTGCCAGAGGATACA

```
  1  MetLysAsnArgAsnArgMetIleValAsnCysValThrAlaSerLeuMetTyrTyrTrp   20
  1  ATGAAAAATAGAAATCGTATGATCGTGAACTGTGTTACTGCTTCCCTGATGTATTATTGC   60
        *         *         *         *         *         *

 21  SerLeuProAlaLeuAlaGluGlnSerSerSerGluIleLysIleValArgAspGluTyr   40
 61  AGCTTACCTGCACTGGCTGAGCAGTCGTCAAGTGAGATAAAGATTGTTCGCCATGAATAC  120
        *         *         *         *         *         *

 41  GlyMetProHisIleTyrAlaAsnAspThrTrpHisLeuPheTyrGlyTyrGlyTyrVal   60
121  GGCATGCCGCATATTTATGCCAATGATACATGGCACCTATTTTATGCCTATGGCTATGTA  180
        *         *         *         *         *         *

 61  ValAlaGlnAspArgLeuPheGlnMetGluMetAlaArgArgSerThrGlnGlyThrVal   80
181  GTAGCACAAGATCGCCTTTTTCAGATGGAAATGGCACGTCGCAGTACTCAAGGGACTGTC  240
        *         *         *         *         *         *

 81  AlaGluValLeuGlyLysAspPheValLysPheAspLysAspIleArgArgAsnTyrTrp  100
241  GCGGAAGTCCTTGGCAAAGATTTTGTGAAATTTGATAAAGATATCCGTCGTAACTACTGG  300
        *         *         *         *         *         *

101  ProAspAlaIleArgAlaGlnIleAlaAlaLeuSerProGluAspMetSerIleLeuGln  120
301  CCGGATGCTATCCGGGCGCAAATTGCTGCCCTTTCCCCAGAGGATATCTCCATTCTGCAA  360
        *         *         *         *         *         *

121  GlyTyrAlaAspGlyMetAsnAlaTrpIleAspLysValAsnThrAsnProGluThrLeu  140
361  GGCTACGCTGATGGAATGAATGCCTGGATTGATAAGGTAAATACCAATCCAGAGACGCTC  420
        *         *         *         *         *         *

141  LeuProLysGlnPheAsnThrPheGlyPheThrProLysArgTrpGluProPheAspVal  160
421  TTACCAAAACAGTTTAATACATTTGGCTTTACTCCTAAGCGCTGGGAACCGTTTGATGTC  480
        *         *         *         *         *         *

161  AlaMetIlePheValGlyThrMetAlaAsnArgPheSerAspSerThrSerGluIleAsp  180
481  GCGATGATATTTGTGGGCACCATGGCAAACCGCTTCTCTGATAGCACTAGCGAAATTGAT  540
        *         *         *         *         *         *

181  AsnLeuAlaLeuLeuThrAlaLeuLysAspLysTyrGlyValSerGlnGlyMetAlaVal  200
541  AATCTGGCACTGCTAACGGCTTTAAAAGATAAATATGGTGTATCACAAGGCATGGCGGTA  600
        *         *         *         *         *         *
```

19

```
201   PheAsnGlnLeuLysTrpLeuValAsnProSerAlaProThrThrIleAlaValGlnGlu   220
601   TTTAATCAGTTGAAATGGCTGGTAAACCCATCAGCGCCAACCACTATTGCCGTACAAGAG   660
         *          *          *          *          *          *

221   SerAsnTyrProLeuLysPheAsnGlnGlnAsnSerGlnThrAlaAlaLeuLeuProArg   240
661   AGTAACTACCCACTTAAATTTAATCAGCAAAACTCGCAAACAGCAGCTCTGTTGCCACGC   720
         *          *          *          *          *          *

241   TyrAspLeuProAlaProMetLeuAspArgProAlaLysGlyAlaAspGlyAlaLeuLeu   260
721   TACGATTTACCTGCACCAATGCTTGACCGACCAGCAAAAGGGGCGGATGGCGCACTGCTG   780
         *          *          *          *          *          *

261   AlaLeuThrAlaGlyLysAsnArgGluThrIleValAlaGlnPheAlaGlnGlyGlyAla   280
781   GCGTTAACAGCAGGGAAGAACCGGGAAACTATTGTTGCACAATTTGCACAGGGTGGTGCC   840
         *          *          *          *          *          *

281   AsnGlyLeuAlaGlyTyrProThrThrSerAsnMetTrpValIleGlyLysSerLysAla   300
841   AATGGTCTGGCGGGGTATCCAACGACCAGCAATATGTGGGTTATCGGCAAAAGCAAAGCC   900
         *          *          *          *          *          *

301   GlnAspAlaLysAlaIleMetValAsnGlyProGlnPheGlyTrpTyrAlaProAlaTyr   320
901   CAGGATGCGAAAGCAATCATGGTAAATGGTCCGCAGTTTGGCTGGTATGCGCCTGCGTAT   960
         *          *          *          *          *          *

321   ThrTyrGlyIleGlyLeuHisGlyAlaGlyTyrAspValThrGlyAsnThrProPheAla   340
961   ACTTATGGTATTGGTCTGCACGGTGCTGGTTATGATGTCACTGGCAATACACCATTTGCC   1020
         *          *          *          *          *          *

341   TyrProGlyLeuValPheGlyHisAsnGlyValIleSerTrpGlySerThrAlaGlyPhe   360
1021  TATCCTGGGCTGGTTTTTGGTCATAATGGTGTGATTTCCTGGGGATCAACGGCAGGTTTC   1080
         *          *          *          *          *          *

361   GlyAspAspValAspIlePheAlaGluArgLeuSerAlaGluLysProGlyTyrTyrLeu   380
1081  GGCGATGATGTCGATATTTTTGCTGAACGGCTGTCGGCAGAGAAACCAGGCTACTACTTG   1140
         *          *          *          *          *          *

381   HisAsnGlyLysTrpValLysMetLeuSerArgGluGluThrIleThrValLysAsnGly   400
1141  CATAATGGTAAGTGGGTGAAAATGTTAAGCCGTGAGGAAACCATTACGGTGAAAAATGGT   1200
         *          *          *          *          *          *

401   GlnAlaGluThrPheThrValTrpArgThrValHisGlyAsnIleLeuGlnThrAspGln   420
1201  CAGGCAGAGACCTTTACTGTCTGGCGTACGGTGCATGGCAACATTCTCCAAACTGACCAG   1260
         *          x          *          x          *          *

421   ThrThrGlnThrAlaTyrAlaLysSerArgAlaTrpAspGlyLysGluValAlaSerLeu   440
1261  ACGACACAAACGGCTTACGCTAAATCCCGCGCATGGGATGGTAAAGAGGTGGCGTCTTTG   1320
         *          *          *          *          *          x
```

20

```
441   LeuAlaTrpThrHisGlnMetLysAlaLysAsnTrpGlnGluTrpThrGlnGlnAlaAla   460
1321  CTGGCCTGGACTCATCAGATGAAGGCCAAAAATTGGCAGGAGTGGACACAGCAGGCAGCG  1380
         *         *         *         *         *         *


461   LysGlnAlaLeuThrIleAsnTrpTyrTyrAlaAspValAsnGlyAsnIleGlyTyrVal   480
1381  AAACAAGCACTGACCATCAACTGGTACTATGCTGATGTAAACGGCAATATTGGTTATGTT  1440
         *         *         *         *         *         *


481   HisThrGlyAlaTyrProAspArgGlnSerGlyHisAspProArgLeuProValProGly   500
1441  CATACTGGTGCTTATCCAGATCGTCAATCAGGCCATGATCCGCGATTACCCGTTCCTGGT  1500
         *         *         *         *         *         *


501   ThrGlyLysTrpAspTrpLysGlyLeuLeuProPheGluMetAsnProLysValTyrAsn   520
1501  ACGGGAAAATGGGACTGGAAAGGGCTATTGCCTTTTGAAATGAACCCTAAGGTGTATAAC  1560
         *         *         *         *         *         *


521   ProGlnSerGlyTyrIleAlaAsnTrpAsnAsnSerProGlnLysAspTyrProAlaSer   540
1561  CCCCAGTCGGGATATATTGCTAACTGGAACAATTCTCCCCAAAAAGATTATCCCGCTTCA  1620
         *         *         *         *         *         *


541   AspLeuPheAlaPheLeuTrpGlyGlyAlaAspArgValThrGluIleAspArgLeuLeu   560
1621  GATCTGTTTGCCTTTTTGTGGGGTGGTGCAGATCGCGTTACGGAGATCGACCGACTGCTT  1680
         *         *         *         *         *         *


561   GluGlnLysProArgLeuThrAlaAspGlnAlaTrpAspValIleArgGlnThrSerArg   580
1681  GAGCAAAAGCCACGCTTAACTGCTGATCAGGCATGGGATGTTATTCGCCAAACCAGTCGT  1740
         *         *         *         *         *         *


581   GlnAspLeuAsnLeuArgLeuPheLeuProThrLeuGlnAlaAlaThrSerGlyLeuThr   600
1741  CAGGATCTTAACCTGAGGCTTTTTTTTACCTACTCTGCAAGCAGCGACATCTGGTTTGACA  1800
         *         *         *         *         *         *


601   GlnSerAspProArgArgGlnLeuValGluThrLeuThrArgTrpAspGlyIleAsnLeu   620
1801  CAGAGCGATCCGCGTCGTCAGTTGGTAGAAACATTAACACGTTGGGATGGCATCAATTTG  1860
         *         *         *         *         *         *


621   LeuAsnAspAspGlyLysThrTrpGlnGlnProGlySerAlaIleLeuAsnValTrpLeu   640
1861  CTTAATGATGATGGTAAAACCTGGCAGCAGCCAGGCTCTGCCATCCTGAACGTTTGGCTG  1920
         *         *         *         *         *         *


641   ThrSerMetLeuLysArgThrValValAlaAlaValProMetProPheAspLysTrpTyr   660
1921  ACCAGTATGTTGAAGCGTACCGTAGTGGCTGCCGTACCTATGCCATTTGATAAGTGGTAC  1980
         *         *         *         *         *         *


661   SerAlaSerGlyTyrGluThrThrGlnAspGlyProThrGlySerLeuAsnIleSerVal   680
1981  AGCGCCAGTGGCTACGAAACAACCCAGGACGGCCCAACTGGTTCGCTGAATATAAGTGTT  2040
         *         *         *         *         *         *


681   GlyAlaLysIleLeuTyrGluAlaValGlnGlyAspLysSerProIleProGlnAlaVal   700
2041  GGAGCAAAAATTTTGTATGAGGCGGTGCAGGGAGACAAATCACCAATCCCACAGGCGGTT  2100
         *         *         *         *         *         *
```

21

```
 701   AspLeuPheAlaGlyLysProGlnGlnGluValValLeuAlaAlaLeuGluAspThrTrp     720
2101   GATCTGTTTGCTGGGAAACCACAGCAGGAGGTTGTGTTGGCTGCGCTGGAAGATACCTGG     2160
            *           *           *           *           *           *


 721   GluThrLeuSerLysArgTyrGlyAsnAsnValSerAsnTrpLysThrProAlaMetAla     740
2161   GAGACTCTTTCCAAACGCTATGGCAATAATGTGAGTAACTGGAAAACACCTGCAATGGCC     2220
            *           *           *           *           *           *


 741   LeuThrPheArgAlaAsnAsnPhePheGlyValProGlnAlaAlaAlaGluGluThrArg     760
2221   TTAACGTTCCGGGCAAATAATTTCTTTGGTGTACCGCAGGCCGCAGCGGAAGAAACGCGT     2280
            *           *           *           *           *           *


 761   HisGlnAlaGluTyrGlnAsnArgGlyThrGluAsnAspMetIleValPheSerProThr     780
2281   CATCAGGCGGAGTATCAAAACCGTGGAACAGAAAACGATATGATTGTTTTCTCACCAACG     2340
            *           *           *           *           *           *


 781   ThrSerAspArgProValLeuAlaTrpAspValValAlaProGlyGlnSerGlyPheIle     800
2341   ACAAGCGATCGTCCTGTGCTTGCCTGGGATGTGGTCGCACCCGGTCAGAGTGGGTTTATT     2400
            *           *           *           *           *           *


 801   AlaProAspGlyThrValAspLysHisTyrGluAspGlnLeuLysMetTyrGluAsnPhe     820
2401   GCTCCCGATGGAACAGTTGATAAGCACTATGAAGATCAGCTGAAAATGTACGAAAATTTT     2460
            *           *           *           *           *           *


 821   GlyArgLysSerLeuTrpLeuThrLysGlnAspValGluAlaHisLysGluSerGlnGlu     840
2461   GGCCGTAAGTCGCTCTGGTTAACGAAGCAGGATGTGGAGGCGCATAAGGAGTCGCAGGAA     2520
            *           *           *           *           *           *


 841   ValLeuHisValGlnArg *                                              860
2521   GTGTTGCACGTTCAGAGATAA                                             2580
            *           *           *           *           *           *
```

22

# FIG.2

Alu I    Eco RV      Alu I   Ava II      Bgl II      Pvu I

Dde I      Dde I    Hpa I   Taq I      Taq I      Hpa I

Bp

0   200   400   600   800   1000   1200   1400   1600   1800   2000   2200   2400   2600

27      235   290      846

Glu Gln Ser   Gln Thr Ala   Ser Asn Met    Aminosäure-position

ATG                   TAA
1   79      705   868      2538 Bp

├── $\alpha$-Untereinheit ──┤    ├────── $\beta$-Untereinheit ──────┤

M G 24133      M G 64655

EP 0 180 225 B1

# FIG.3

# FIG.4

```
                                     Eco RI
        Hind III  Dde I    Eco RV  |Dde I        Hind II
        ........ ▮▮▮▮▮▮▮▮▮▮▮▮▮▮▮▮▮▮▮▮▮▯▯▯▯▯▯▯▯▯▯▯▯▯▯▯▯▯▯▯▯▯ ⌐⌐⌐⌐⌐ __pBT 142
        pBR 322        Pen-G                      Lac Z
```

Hind III, Hind II

isoliere 800 Bp-Fragment

    Dde I

isoliere 480 Bp-Fragment

    Glu  Gln  Ser
5' TGAG CAG TCG . . . . 3'
3'     C GTC AGC . . . . 5'

    Klenow Pol I
    dTTP, dCTP

5' TGAG CAG TCG . . . . 3'
3' CTC GTC AGC . . . . 5'

    S1 Nuklease

**Eco-ATG-Linker**      Glu  Gln  Ser
5' CATG GAA TTC ATG 3'   5' GAG CAG TCG . . . . 3'
3' GTAC CTT AAG TAC 5'   3' CTC GTC AGC . . . . 5'

    T₄ Ligase

5' CATG GAA TTC ATG GAG CAG TCG . . . . 3'
3' GTAC CTT AAG TAC CTC GTC AGC . . . . 5'

    Eco RI

isoliere 270 Bp-Fragment

# FIG.5

Bam HI ~2,9 Kb
pKK 177-3

Eco RI
Sma I
Bam HI
Sal I
Pst I
Hind III

tac
T₁ T₂
 Apʳ
Pvu I

Lac Z

~8,3 Kb
pBT 117

Eco RI
Bam HI

Eco RI
Lac Y

Kmʳ
pACYC 177

Pst

| Eco RI
| Pst I
isoliere 2,9 kb Fragment

| Eco RI partial.
| Pst I
isoliere 5,5 kb Fragment

+270 Bp Eco RI Fragment

5ˡ AATTC ATG GAG CAG TCG . . . . 3ˡ
3ˡ          G TAC CTC GTC AGC . . . . 5ˡ

| T₄ Ligase

pKK 177-3

Pvu I  Hind III
Apʳ   Pst I
T₁ T₂

~8,5 Kb
pBT II/3

Lac Y

Eco RI

Pen-G

Eco RI
Eco RV
Eco RI

Lac Z

# FIG.6

pKK 177-3

Hind III
Pst I

$Ap^r$ $T_2$ $T_1$

~ 8,5 Kb
pBT II/3

Lac Y

Eco RI

Eco RI
Eco RV
Eco RI

Pen-G

Lac Z

Eco RI
Lac
PO
Hind III
Eco RV

~ 7,8 Kb
pBT E1-11

$Ap^r$

Hpa I
Ava II

Pen G

Hpa I
Ava I

$Tc^r$

Eco RI
Hind III

Hind III, Klenow Pol I
+4d NTP's

Eco RV

isoliere 3.1kb-Fragment

Ava I, Klenow Pol I
+4d NTP's

Eco RV

isoliere 2,5 kb Fragment

$T_4$ Ligase

pKK 177-3

$Ap^r$ $T_2$ $T_1$

~5,6 Kb
pBT 212

PO
Eco RI
Eco RV

Pen-G

27

# FIG.7

# FIG.8

Eco RI
Lac
Po
Hind III

~ 7,8 Kb
pBT E1-11
Ap$^r$

Hpa I
Ava II

Hpa I
Ava I

Eco RI
Hind III

Tc$^r$

Bam HI
~ 2,9Kb
pKK 177-3

Eco RI
Sma I
Bam HI
Sal I
Pst I
Hind III

tac

T$_1$ T$_2$

Ap

Pvu I

|  |  |  |  |
|---|---|---|---|
| Hind III | | Hind III | |
| Eco RI | | Ava II | |

isoliere 2.9 kb-Fragment    isoliere 2.5 kb-Fragment

Ava II
↓

5' AATTC ATG AGC AAT ATG TGG GTT . . . . . . . 3'
3'        G TAC TCG TTA TAC ACC CAA . . . . . . 5'

**60 Bp-Fragment**

↓ T$_4$ Ligase

~ 5.5 Kb Ap$^r$
pBT 1000  T$_2$
T$_1$  Hind III
Eco

PO Ava II

Eco RI

FIG.9